(19) Europäisches Patentamt
European Patent Office
Office européen des brevets

(11) EP 2 428 575 A1

(12) # EUROPÄISCHE PATENTANMELDUNG

(43) Veröffentlichungstag:
**14.03.2012 Patentblatt 2012/11**

(21) Anmeldenummer: **11189707.0**

(22) Anmeldetag: **20.07.2006**

(51) Int Cl.:
***C12P 7/02*** *(2006.01)* ***C12N 9/02*** *(2006.01)*
***C12P 41/00*** *(2006.01)*

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **27.07.2005 AT 12612005**

(62) Dokumentnummer(n) der früheren Anmeldung(en) nach Art. 76 EPÜ:
**06776315.1 / 1 907 556**

(71) Anmelder: **IEP GmbH**
**65203 Wiesbaden (DE)**

(72) Erfinder:
- **Tschentscher, Anke**
  **65347 Eltville - Hattenheim (DE)**
- **Gupta, Antje**
  **65207 Wiesbaden (DE)**
- **Bobkova, Maria**
  **65207 Wiesbaden (DE)**

(74) Vertreter: **Schwarz & Partner**
**Patentanwälte**
**Wipplingerstraße 30**
**1010 Wien (AT)**

Bemerkungen:
Diese Anmeldung ist am 18-11-2011 als Teilanmeldung zu der unter INID-Code 62 erwähnten Anmeldung eingereicht worden.

(54) **Oxidoreduktasen zur stereoselektiven Reduktion von Ketoverbindungen**

(57) Die Erfindung betrifft ein Verfahren zur enantioselektiven enzymatischen Reduktion einer Ketoverbindung zur entsprechenden chiralen Hydroxyverbindung, wobei die Ketoverbindung mit einer Oxidoreduktase in Gegenwart eines Co-Faktors reduziert wird und ist dadurch gekennzeichnet, dass eine Oxidoreduktase eingesetzt wird, für welche (a) die Nukleinsäuresequenz gemäß SEQ ID NO: 11,15 oder 130 kodiert oder (b) elne Nukleinsäuresequenz, deren komplementärer Strang mit den in (a) genannten Nukleinsäuresequenzen unter hochstringenten Bedingungen hybridisiert.

EP 2 428 575 A1

SEQUENCE LISTING

<110> IEP GmbH

<120> Oxidoreduktasen zur stereoselektiven Reduktion von Ketoverbindungen

<130> I 11029C

<140> A 1261/2005
<141> 2005-07-27

<160> 130

<170> PatentIn version 3.3

<210> 1
<211> 282
<212> PRT
<213> Rhodotorula mucilaginosa

<400> 1

```
Met Pro Ala Thr Leu Arg Leu Asp Lys Lys Val Ala Ile Ile Thr Gly
1               5                   10                  15

Gly Ala Ser Gly Ile Gly Leu Glu Ser Ala Leu Val Phe Ala Gly Glu
            20                  25                  30

Gly Ala His Val Val Val Ala Asp Ile Asn Val Glu Ala Ala Asn Arg
        35                  40                  45

Ala Val Glu Ile Ile Lys Thr Gln Val Gln Asp Ala Pro Lys Ala Ile
    50                  55                  60

Ala Val Lys Cys Asp Val Ser Lys Glu Asp Asp Ile Lys Asn Leu Val
65                  70                  75                  80

Ala Thr Ala Val Glu Thr Phe Gly Arg Leu Asp Val Met Phe Asn Asn
                85                  90                  95

Ala Gly Ile Met His Pro Glu Asp Asp Asn Ala Leu Asn Thr Ser Glu
            100                 105                 110

Arg Ile Trp Asp Leu Thr Met Asn Ile Asn Val Lys Gly Val Trp Trp
        115                 120                 125

Gly Cys Lys Tyr Ala Ile Asp Ala Met Arg Lys Asn Pro Gly Gly Ser
    130                 135                 140

Lys Gly Ser Ile Ile Asn Thr Ala Ser Phe Val Ala Ile Leu Gly Ala
145                 150                 155                 160

Ala Thr Pro Gln Ile Ala Tyr Thr Ala Ser Lys Gly Ala Val Leu Ala
                165                 170                 175
```

Met Thr Arg Glu Leu Ala Met Val His Ala Arg Glu Gly Ile Arg Ile
180 185 190

Asn Ser Leu Cys Pro Gly Pro Leu Lys Thr Glu Leu Leu Met Lys Phe
195 200 205

Leu Asp Thr Pro Glu Lys Lys Glu Arg Arg Met Val His Ile Pro Met
210 215 220

Gly Arg Phe Gly Glu Ala Val Glu Gln Ala Arg Ala Ala Ala Phe Leu
225 230 235 240

Ala Ser Asp Asp Ser Ser Phe Ile Thr Gly Thr Asp Phe Lys Val Asp
245 250 255

Gly Gly Ile Ser Ser Cys Tyr Val Thr Pro Glu Gly Glu Gln Ala Leu
260 265 270

Ala Ala Pro Ser Asn Leu Ala Pro Lys Ala
275 280

<210> 2
<211> 254
<212> PRT
<213> Pichia farinosa

<400> 2

Met Ala Tyr Asn Phe Thr Asn Lys Val Ala Ile Ile Thr Gly Gly Ile
1 5 10 15

Ser Gly Ile Gly Leu Ala Thr Val Glu Lys Phe Ala Lys Leu Gly Ala
20 25 30

Lys Val Val Ile Gly Asp Ile Gln Lys Glu Glu Tyr Lys Glu Ala Ala
35 40 45

Phe Thr Asn Leu Lys Asn Lys Gly Ile Asn Leu Asp Gln Leu Thr Tyr
50 55 60

Val His Thr Asp Val Thr Ala Asn Ser Ala Asn Glu Asn Leu Leu Lys
65 70 75 80

Thr Ala Ile Ser Ser Phe Gly Gly Val Asp Phe Val Val Ala Asn Ser
85 90 95

Gly Ile Ala Lys Asp Gln Arg Ser Glu Glu Met Thr Tyr Glu Asp Phe
100 105 110

Lys Lys Ile Ile Asp Val Asn Leu Asn Gly Val Phe Ser Leu Asp Lys
        115                 120                 125

Leu Ala Ile Asp Tyr Trp Leu Lys Asn Lys Lys Lys Gly Ser Ile Val
    130                 135                 140

Asn Thr Gly Ser Ile Leu Ser Phe Val Gly Thr Pro Gly Leu Ser His
145                 150                 155                 160

Tyr Cys Ala Ser Lys Gly Gly Val Lys Leu Leu Thr Gln Thr Leu Ala
                165                 170                 175

Leu Glu Gln Ala Lys Asn Gly Ile Arg Val Asn Cys Ile Asn Pro Gly
            180                 185                 190

Tyr Ile Arg Thr Pro Leu Leu Glu Phe Leu Pro Lys Asp Lys Tyr Asp
        195                 200                 205

Ala Leu Val Asp Leu His Pro Met Gly Arg Leu Gly Glu Pro Glu Glu
    210                 215                 220

Ile Ala Asn Ala Ile Ala Phe Leu Val Ser Asp Glu Ala Ser Phe Ile
225                 230                 235                 240

Thr Gly Thr Thr Leu Leu Val Asp Gly Gly Tyr Thr Ala Gln
                245                 250

<210> 3
<211> 336
<212> PRT
<213> Pichia stipitis

<400> 3

Met Ser Ile Pro Ala Thr Gln Tyr Gly Phe Val Phe Thr Lys Lys Asp
1               5                   10                  15

Gly Leu Lys Ile Arg Glu Asn Met Pro Val Leu Glu Pro Lys Ala Asp
            20                  25                  30

Gln Val Leu Leu Lys Val Asp Ala Val Gly Leu Cys His Ser Asp Leu
        35                  40                  45

His Ala Ile Tyr Asp Gly Phe Asp Phe Gly Asp Asn Tyr Val Met Gly
    50                  55                  60

His Glu Ile Ala Gly Thr Ile Val Lys Lys Gly Ala Met Val Asp Phe
65                  70                  75                  80

Trp Asp Leu Asn Thr Arg Val Ala Cys Phe Gly Pro Asn Ser Cys Gly
                85                  90                  95

```
His Cys Gln Leu Cys Arg Thr Gly Phe Glu Asn Asp Cys Ile Asn Val
            100                 105                 110

Val Asn Gly Trp Phe Gly Leu Gly Lys Asn Gly Gly Tyr Gln Gln Tyr
        115                 120                 125

Leu Leu Val Glu Lys Pro Arg Asn Leu Val Ala Ile Pro Asp Asn Val
    130                 135                 140

Glu Leu Ser Asp Ala Ala Ala Ile Thr Asp Ala Leu Leu Thr Pro Tyr
145                 150                 155                 160

His Ala Met Arg Leu Ala Gly Val Arg Ser Gly Thr Lys Leu Leu Gln
                165                 170                 175

Ile Gly Ala Gly Gly Leu Gly Val Asn Gly Ile Gln Ile Ala Lys Ala
            180                 185                 190

Phe Gly Ala Gln Val Thr Val Ile Asp Lys Lys Pro Glu Ala Val Asp
        195                 200                 205

Val Ala Lys Ser Leu Gly Ala Asp Glu Val Tyr Ser Ala Leu Pro Glu
    210                 215                 220

Ser Thr Ser Pro Gly Ser Phe Asp Val Ala Ile Asp Tyr Val Ser Thr
225                 230                 235                 240

Gln Gly Thr Phe Asp Thr Cys Gln Lys Tyr Val Arg Ser Lys Gly Asn
                245                 250                 255

Ile Val Pro Val Gly Leu Ala Ala Pro Arg Ile Ser Phe Asn Leu Gly
            260                 265                 270

Asp Leu Ala Leu Arg Glu Ile Asn Val Leu Gly Ser Phe Trp Gly Thr
        275                 280                 285

Ser Ser Asp Leu Lys Glu Cys Phe Asp Leu Val Ser Lys Gly Lys Val
    290                 295                 300

Lys Pro Lys Val Thr Val Ala Pro Leu Lys Gln Leu Pro Glu Tyr Ile
305                 310                 315                 320

Val Lys Leu Gln Asn Ser Ala Tyr Glu Gly Arg Val Val Phe Lys Pro
                325                 330                 335

<210>  4
<211>  252
<212>  PRT
```

<213> Leuconostoc carnosum

<400> 4

```
Met Thr Asp Arg Leu Lys Asn Lys Val Ala Ile Ile Thr Gly Gly Thr
1               5                   10                  15

Leu Gly Ile Gly Leu Ala Met Ala Gln Lys Phe Val Glu Glu Gly Ala
            20                  25                  30

Lys Val Val Ile Thr Gly Arg Arg Ala Asn Val Gly Ala Glu Ala Leu
        35                  40                  45

Lys Thr Ile Gly Asp Glu Ser Val Ala Arg Phe Val Gln His Asp Ala
    50                  55                  60

Ser Asp Glu Lys Gly Trp Ile Asp Leu Phe Glu Asn Thr Ile Lys Trp
65                  70                  75                  80

Phe Gly His Val Asp Thr Val Val Asn Asn Ala Gly Val Ala Ile Ala
                85                  90                  95

Lys Asn Ile Glu Glu Thr Thr Tyr Glu Asp Trp Lys Phe Leu Gln Ser
            100                 105                 110

Ile Asn Ser Asp Gly Val Phe Leu Gly Thr Lys Tyr Gly Met Gln Tyr
        115                 120                 125

Met Lys Asn Gln Ala Gly Gly Ala Ser Ile Ile Asn Met Ser Ser Ile
    130                 135                 140

Glu Gly Phe Val Gly Asp Pro Asn Leu Ala Ala Tyr Asn His Ser Lys
145                 150                 155                 160

Gly Gly Val Arg Ile Leu Ser Lys Ser Ala Ala Leu His Ala Ala Leu
                165                 170                 175

Asn Asp Tyr Asn Leu Arg Val Asn Thr Ile His Pro Gly Tyr Ile Lys
            180                 185                 190

Thr Pro Leu Val Asp Gly Ile Asp Gly Ala Glu Glu Ala Gln Ser Gln
        195                 200                 205

Arg Thr Gln Thr Pro Met Gly His Ile Gly Glu Pro Asn Asp Ile Ala
    210                 215                 220

Tyr Met Ala Val Tyr Leu Ala Ser Glu Glu Ser Lys Phe Ala Thr Gly
225                 230                 235                 240

Ala Glu Phe Val Val Asp Gly Gly Tyr Leu Ala Gln
```

245 250

<210> 5
<211> 347
<212> PRT
<213> Microbacterium sp.

<400> 5

Met Lys Ala Leu Gln Tyr Thr Lys Ile Gly Ser His Pro Glu Val Val
1 5 10 15

Glu Ile Glu Lys Pro Ser Pro Gly Pro Gly Gln Val Leu Leu Lys Val
20 25 30

Thr Ala Ala Gly Val Cys His Ser Asp Glu Phe Val Met Ser Leu Ser
35 40 45

Glu Glu Gln Tyr Thr Ala Ala Gly Tyr Pro Leu Pro Leu Thr Leu Gly
50 55 60

His Glu Gly Ala Gly Ile Val Glu Glu Leu Gly Glu Gly Val Glu His
65 70 75 80

Leu Ser Val Gly Asp Ala Val Ala Val Tyr Gly Pro Trp Gly Cys Gly
85 90 95

Arg Cys Arg Asn Cys Ala Gln Gly Lys Glu Asn Tyr Cys Thr Asn Ala
100 105 110

Gln Ala Glu Gly Ile Met Pro Pro Gly Leu Gly Ala Pro Gly Ser Met
115 120 125

Ala Glu Tyr Met Ile Val Asp Ser Ala Arg His Leu Val Pro Leu Gly
130 135 140

Asp Leu Asp Pro Val Gln Asn Val Ser Leu Thr Asp Ala Gly Leu Thr
145 150 155 160

Pro Tyr His Ala Val Lys Thr Ser Leu Pro Lys Leu Gly Ala Gly Thr
165 170 175

Thr Ala Val Val Ile Gly Thr Gly Gly Leu Gly His Val Ala Ile Gln
180 185 190

Ile Leu Arg Ala Val Ser Ala Ala Thr Val Ile Ala Leu Asp Val Asn
195 200 205

Asp Glu Lys Leu Ala Leu Ala Lys Glu Val Gly Ala His His Thr Val
210 215 220

Met Ser Asp Gly Gly Ala Val Asp Ala Ile Arg Arg Leu Thr Asp Gly
225                 230                 235                 240

Leu Gly Ala Asn Ala Val Phe Asp Phe Val Gly Ala Asp Pro Thr Ile
                245                 250                 255

Ala Thr Ala Ile Gly Ala Ala Ala Leu Asp Ala Asp Ile Thr Ile Val
            260                 265                 270

Gly Ile Gly Gly Gly Thr Ala His Val Gly Phe Gly Thr Val Ala Tyr
        275                 280                 285

Asp Ala Ala Leu Arg Ile Pro Tyr Trp Gly Ser Arg Ser Glu Leu Ile
    290                 295                 300

Glu Val Leu Asp Leu Ala Arg Ser Gly Gln Val Gly Val Glu Ile Gln
305                 310                 315                 320

Arg Tyr Ser Leu Asp Asp Gly Pro Lys Ala Tyr Glu Ala Leu Ala Ala
                325                 330                 335

Gly Thr Val Arg Gly Arg Ala Val Ile Val Pro
            340                 345

<210> 6
<211> 347
<212> PRT
<213> Gordona rubropertinctus

<400> 6

Met Lys Ala Ile Gln Ile Ile Gln Pro Gly Lys Pro Pro Glu Leu Arg
1               5                   10                  15

Glu Val Glu Lys Pro Thr Pro Arg Pro Gly Gln Val Leu Leu Lys Val
            20                  25                  30

Thr Ala Ala Gly Ala Cys His Ser Asp Asp Phe Val Leu Asn Leu Pro
        35                  40                  45

Glu Glu Gly Phe Pro Tyr Pro Leu Pro Met Thr Leu Gly His Glu Gly
    50                  55                  60

Ala Gly Val Val Ala Glu Val Gly Thr Gly Val Thr Gly Ile Ser Glu
65                  70                  75                  80

Gly Thr Ser Val Ala Val Tyr Gly Ala Trp Gly Cys Gly Val Cys His
                85                  90                  95

Phe Cys Ala Arg Gly Leu Glu Asn Tyr Cys Ser Arg Ala Gly Glu Leu

100 105 110

Gly Ile Thr Pro Pro Gly Leu Gly Asn Pro Gly Ala Met Ala Glu Tyr
115 120 125

Leu Leu Val Asp Asp Ala Arg His Leu Val Pro Leu Gly Asp Leu Asp
130 135 140

Pro Val Ala Ala Val Pro Leu Thr Asp Ala Gly Leu Thr Pro Tyr His
145 150 155 160

Ala Ile Lys Pro Ser Leu Pro Lys Leu Val Gly Gly Thr Thr Ala Val
165 170 175

Val Ile Gly Ala Gly Gly Leu Gly His Val Gly Ile Gln Leu Leu Arg
180 185 190

His Leu Thr Pro Ser Arg Val Ile Ala Leu Asp Val Ser Asp Asp Lys
195 200 205

Leu Ala Phe Ala Arg Glu Val Gly Ala His Glu Val Val Leu Ser Asp
210 215 220

Ala Asp Ala Val Ala Asn Val Arg Lys Ile Thr Gly Asn Asp Gly Ala
225 230 235 240

Thr Ala Val Phe Asp Phe Val Gly Leu Gln Pro Thr Leu Asp Ile Ala
245 250 255

Met Gly Val Val Gly Thr Met Gly Asp Val Val Ile Val Gly Ile Gly
260 265 270

Asp Met Val Ala Thr Ala Lys Val Gly Phe Phe Thr Gln Pro Tyr Glu
275 280 285

Val Ser Val Arg Ala Pro Tyr Trp Gly Ala Arg Asp Glu Leu Ile Glu
290 295 300

Val Leu Asp Leu Ala Arg Asp Gly Val Leu Glu Val Ala Val Glu Arg
305 310 315 320

Phe Ser Leu Asp Asp Gly Val Glu Ala Tyr Arg Arg Leu Ala Ala Asn
325 330 335

Asp Leu Arg Gly Arg Ala Val Val Val Pro Asp
340 345

<210> 7
<211> 339

<212> PRT
<213> Pichia trehalophila

<400> 7

Met Cys Thr Ser Gln Ser Gly Tyr Val Tyr His Ser Gly Arg Pro Leu
1               5                   10                  15

Leu Thr Lys Glu Glu Leu Ser Ile Pro Glu Pro Lys Gly Ser Glu Ile
            20                  25                  30

Val Leu Lys Val Arg Ala Ala Gly Leu Cys Ser Ser Asp Val His Val
        35                  40                  45

Leu Asn Ser Ser Leu Pro Leu Thr Tyr Pro Asn Ser Phe Ala Met Gly
    50                  55                  60

His Glu Ile Ala Gly Glu Ile Tyr Lys Leu Gly Pro Asn Val Asp Ala
65                  70                  75                  80

Asp Lys Tyr Ser Ile Gly Asp Gly Tyr Ala Val His Gly Leu Asn Ser
                85                  90                  95

Cys Gly Asp Cys Ser Phe Cys Lys Val Gly Ser Gln Asn Leu Cys Thr
            100                 105                 110

Asp Asn Asn Ser Thr Trp Tyr Gly Leu Gly Lys Asn Gly Gly Tyr Glu
        115                 120                 125

Gln Tyr Val Leu Val Lys Ser Val His Asp Leu Ile Lys Ile Pro Glu
    130                 135                 140

Gly Val Ser Phe Ser Glu Ala Ala Val Ala Ser Asp Ala Val Leu Thr
145                 150                 155                 160

Pro Tyr His Ala Ile Ser Thr Cys Asn Leu Lys Ala Thr Ser Lys Val
                165                 170                 175

Leu Val Ile Gly Cys Gly Gly Leu Gly Thr Cys Ala Leu Gln Ile Ile
            180                 185                 190

Lys Leu Tyr Ser Ala Tyr Val Val Cys Val Asp Ser Lys Ala Glu Leu
        195                 200                 205

Glu Glu Leu Ala Lys Glu Tyr Gly Ala Asp Glu Phe Tyr Thr Asp Leu
    210                 215                 220

Ser Lys Ser Ser Val Pro Lys Met Ser Phe Asp Cys Val Phe Asp Phe
225                 230                 235                 240

Val Ala Ile Gln Pro Thr Phe Thr Ile Ser Gln Asn Tyr Val Lys Ser
245 250 255

Gly Gly Ile Ile Lys Pro Val Gly Leu Gly Ala Pro Ser Leu Thr Phe
260 265 270

Ser Leu Leu Asp Leu Gly Cys Arg Asp Val Lys Ile Ile Gly Ser Phe
275 280 285

Trp Gly Thr Gln Ala Glu Gln Lys Asp Cys Met Glu Leu Ile Gln Arg
290 295 300

Gly Leu Val Lys Pro Leu Ile Thr Ser Phe Thr Phe Asp Glu Phe Pro
305 310 315 320

Gln Ala Tyr Glu Leu Leu Ser Thr Gly Lys Ser Lys Gly Arg Leu Val
325 330 335

Ile Ser Gln

<210> 8
<211> 254
<212> PRT
<213> Candida nemodendra

<400> 8

Met Gly Tyr Asn Leu Ile Asn Lys Val Ala Val Val Thr Gly Gly Cys
1 5 10 15

Ser Gly Ile Gly Leu Ala Val Thr Lys Lys Tyr Leu Glu Leu Gly Ala
20 25 30

Lys Val Val Ile Gly Asp Val Ser Thr Lys Glu Lys Phe Asn Glu Val
35 40 45

Ser Ser Glu Leu Lys Val Ala Gly Leu Asn Val Asn Asn Leu Asn Phe
50 55 60

Val Ser Ala Asp Ser Ser Lys Glu Asp Asp Asn Lys Arg Leu Val Asp
65 70 75 80

Glu Ala Ile Lys Asn Phe Gly Gly Leu Asp Ile Val Cys Ala Asn Ala
85 90 95

Gly Ile Gly Ser Met Ile Pro Phe His Glu Met Thr Phe Glu Ala Trp
100 105 110

Arg Lys Leu Leu Ala Val Asn Leu Asp Gly Val Phe Leu Leu Asp Arg
115 120 125

Phe Ala Ile Asp Tyr Trp Leu Lys Asn Ser Lys Pro Gly Val Ile Val
    130                 135                 140

Asn Met Gly Ser Ile His Ser Phe Val Ala Ala Pro Gly Leu Ala His
145                 150                 155                 160

Tyr Ser Ala Ser Lys Gly Gly Val Lys Leu Leu Thr Glu Ala Leu Ala
                165                 170                 175

Leu Glu Tyr Ser Ser Lys Gly Ile Arg Val Asn Ser Val Asn Pro Ala
            180                 185                 190

Tyr Ile Gln Thr Ser Leu Leu Glu Phe Leu Pro Glu Asp Lys Met Asn
        195                 200                 205

Ala Leu Lys Ala Val His Pro Ile Gly Arg Leu Gly Lys Pro Glu Glu
    210                 215                 220

Val Ala Asn Ala Val Ala Phe Leu Ser Ser Asp Glu Ala Thr Phe Ile
225                 230                 235                 240

His Gly Thr Ser Leu Leu Val Asp Gly Gly Tyr Thr Ala Gln
                245                 250

<210> 9
<211> 849
<212> DNA
<213> Rhodotorula mucilaginosa

<220>
<221> gene
<222> (1)..(849)

<400> 9
atgcctgcca ctctccgcct cgacaagaag gttgccatca tcaccggagg agcatccggg 60

atcggcctcg agtcggccct cgtctttgcc ggagaaggcg cccacgtcgt cgtcgccgac 120

atcaacgtcg aagctgccaa ccgcgccgtc gagatcatca agacccaggt tcaggacgcc 180

cccaaggcga tcgccgtcaa gtgcgacgtc tccaaggagg acgacatcaa gaacctcgtc 240

gcgactgctg tcgaaacttt tggcaggctc gatgtcatgt tcaacaacgc cggcatcatg 300

cacccccgagg acgacaatgc gctcaacacc tcggagcgca tctgggacct gaccatgaac 360

attaacgtca agggagtctg gtggggctgc aagtacgcga tcgacgccat gcgcaagaac 420

ccgggcggca gcaaggggag catcatcaac acggcttcgt tcgtcgccat cctcggagcg 480

gcgacgcctc agatcgcata caccgcttcg aagggtgccg tcttggccat gactcgcgag 540

ctcgccatgg ttcacgcgcg cgaagggatc cgaatcaact cgctctgccc cggtccgctc 600

aagacagagc tcctgatgaa gttcctcgac acgccggaga agaaggagcg ccggatggtg 660

cacatcccga tgggtcgctt cggtgaggcg gttgagcagg ctcgcgcggc cgcgttcctc 720

gctagcgacg acagcagctt catcaccgga actgacttca aggtcgacgg cggtatcagc 780

tcgtgctacg tcacgccgga gggcgagcag gccctcgcgg cgccgtccaa cttggctccc 840

aaggcgtag 849

<210> 10
<211> 765
<212> DNA
<213> Pichia farinosa

<220>
<221> gene
<222> (1)..(765)

<400> 10
atggcctata acttcactaa caaagtcgct atcattacag gaggaatttc cggtattggt 60

ttagctacag tcgagaaatt cgctaagttg ggtgctaaag tcgtcatagg agacattcaa 120

aaagaagaat ataaagaagc tgcttttaca aatttgaaga acaaaggaat taatcttgat 180

caattgacgt atgtccacac ggacgtcacc gcaaattcgg caaatgagaa ccttttaaag 240

actgctataa gctcctttgg tggcgttgac tttgtcgtag caaactctgg aatagcaaaa 300

gatcaacgtt ctgaagagat gacttatgaa gatttcaaga aaataatcga tgtcaactta 360

aacggtgttt tttccttgga taagctagca attgattatt ggttaaaaaa taagaaaaag 420

ggctctattg tcaacacggg atctattctt tcatttgttg gtacccccgg gttatcacat 480

tattgtgcgt caaagggtgg agtgaagtta ttgacacaaa ccttggctct cgaacaggct 540

aagaatggca taagagtgaa ttgtataaat cctggttata taagaacacc tttattagag 600

tttttgccta aggacaagta tgacgcttta gtggatcttc atccaatggg tagattaggt 660

gaacctgagg aaattgccaa tgccattgca ttcctcgtct ctgacgaagc gagcttcata 720

actggtacta ctctactcgt tgatggagga tatacagccc agtaa 765

<210> 11
<211> 1011
<212> DNA
<213> Pichia stipitis

<220>
<221> gene
<222> (1)..(1011)

<400> 11
atgtctattc ctgctacaca atatggtttc gtcttcacca aaaaggacgg tttaaaaatt 60

cgcgagaaca tgcctgttct cgaacccaag gctgaccaag tcttgcttaa agtcgacgca 120

gtaggattgt gtcactctga ccttcatgcc atctacgacg gcttcgactt tggtgacaat 180

tacgttatgg gccacgaaat cgccggcacc attgtcaaga agggagccat ggtcgacttt 240

tgggacctaa acacccgtgt tgcctgtttt ggtccaaact cctgtggcca ttgtcaactt 300

tgtcgtactg gttttgaaaa tgattgtatc aatgtcgtca acggctggtt tggattaggt 360

aaaaacggag gctaccagca atatttgttg gttgaaaagc ctcgtaattt ggttgctatt 420

ccagacaacg tcgagctgtc cgatgcagct gccattaccg acgctttgtt gaccccctac 480

catgccatga gattagctgg tgttagatca ggcacgaagc tcttgcaaat tggtgctgga 540

ggattgggag taaatggtat tcagattgct aaagcatttg gagctcaagt cactgttatc 600

gacaaaaagc ccgaggctgt agacgtcgct aagagcctag gcgcagatga agtatattct 660

gcacttcctg aatcaaccag tccgggaagt ttcgatgttg ctatcgacta cgtttctact 720

caaggcactt tcgacacttg tcaaaagtac gtcagatcta agggtaatat tgttcccgtt 780

ggattggccg ctccaagaat ttcgtttaac ttgggagatt tggcccttag agaaattaat 840

gtccttggta gcttctgggg tacatcatcc gacttgaagg aatgtttcga tttggtcagc 900

aagggcaaag tcaaacctaa ggtgactgtt gctccattga agcaattgcc tgaatacatt 960

gtcaagttac agaattcggc ctacgaaggt agagtcgtgt tcaagccatg a 1011

<210> 12
<211> 759
<212> DNA
<213> Leuconostoc carnosum

<220>
<221> gene
<222> (1)..(759)

<400> 12
atgacagatc ggttaaagaa taaagttgct attatcactg gtggtacact tggtattggc 60

ttagcaatgg ctcaaaagtt tgtagaagaa ggcgctaaag ttgtcattac tgggcgtcgt 120

gctaatgttg gtgcagaagc gctaaagaca attggtgatg aatcagtagc acgatttgtt 180

caacatgatg catctgatga aaaaggctgg attgatttat ttgaaaatac gattaaatgg 240

tttggtcatg tcgatacggt tgtcaataat gccggtgttg caattgctaa aaacattgaa 300

gagacaacat atgaagactg gaaatttttg caatcaatca actctgatgg cgttttctta 360

ggaactaagt acggtatgca atatatgaaa aaccaagctg gtggtgcctc aattattaat 420

atgtcatcta ttgaaggatt tgttggtgat cctaacttag ctgcttataa tcattcaaaa 480

ggtggtgtcc gcattttgag taagtcagct gcactacatg cagcattgaa tgactataac 540

ttacgtgtca acacgattca cccaggatat atcaaaacac cattggttga tggtattgat 600

ggtgcagaag aagcccaatc acaaagaact caaacaccta tgggacatat tggtgaacct 660

aatgatattg catatatggc agtctattta gctagtgaag aatcaaagtt tgcaacaggt 720

gctgaattcg ttgttgatgg cggctatttg gcacaataa 759

<210> 13
<211> 1044
<212> DNA
<213> Microbacterium sp.

<220>
<221> gene
<222> (1)..(1044)

<400> 13
atgaaggcac tccagtacac gaagatcgga tcccaccccg aagtcgtcga gatcgagaag 60

ccctcgccgg gtcccgggca ggtactgctc aaagtcaccg ccgccggcgt ctgccactcg 120

gacgagttcg tgatgagcct cagcgaggag cagtacaccg ctgccggcta ccccctgccg 180

ctcaccctcg ggcacgaagg cgccggcatc gtcgaggagc tcggcgaagg tgtcgagcac 240

ctgagcgtcg gagacgccgt cgccgtctac ggcccctggg gttgcggccg ctgccgcaac 300

tgcgcgcagg gcaaggagaa ctactgcacg aacgcccagg cggaggggat catgcctccc 360

ggtctcgggg ctcccggctc aatggcggag tacatgatcg tcgacagcgc gcgacacctc 420

gttccgctcg gcgacctcga ccccgtgcag aacgtttcct tgacggatgc cggcctgacc 480

ccgtaccacg cggtcaagac gtcacttccg aagctgggcg ccggaacgac ggcggtcgtg 540

atcggcaccg gggtctcgg acacgtcgcg attcagatcc tgcgggcggt gtcggccgcg 600

accgtgatcg cgttggacgt caacgacgag aaactcgcgc tggccaagga ggtcggcgcc 660

catcacaccg tcatgagcga cggcggcgcc gtcgacgcga ttcgccggct caccgacggt 720

ctgggcgcga acgccgtctt cgacttcgtc ggtgcggacc cgacgatcgc gacggcgata 780

ggagcagccg cgctcgacgc agacatcacg atcgtcggca tcggcggcgg aacggctcac 840

gtcggtttcg gcaccgtcgc ttatgacgcg gcgcttcgca tcccgtattg ggctcgcgc 900

agcgaactga tcgaggtgct cgacctcgcg cgctcagggc aggtgggagt cgagatccag 960

cgctactcac tcgacgacgg cccgaaggcg tacgaggcgc tcgccgcggg cacggtccgc 1020

ggccgcgccg tcatcgtccc ctga 1044

<210> 14
<211> 1044
<212> DNA
<213> Gordona rubropertinctus

<220>
<221> gene
<222> (1)..(1044)

<400> 14
atgaaggcca ttcagatcat ccagccgggc aaaccgccgg agctgcgcga ggtcgagaaa 60

cccacgccgc gtcccgggca ggtgttgctg aaggtgacgg cagccggcgc ctgccattcg 120

gacgacttcg tcctcaacct gcccgaggaa ggattcccct atcccctgcc gatgacgctc 180

ggccacgaag gggccggcgt ggtcgccgag gtcggtaccg gcgtcaccgg catctccgag 240

ggcacctcgg tggccgtgta cggagcctgg ggttgcggcg tctgtcactt ctgcgcccgc 300

ggcctggaga actactgcag ccgagccggc gaactcggca tcaccccacc gggtctcggc 360

aacccgggcg cgatggccga gtacctgctc gtggacgacg cacggcatct ggtgccgctc 420

ggtgacctcg acccggtggc tgcagtccca ctcaccgatg ccggcctcac gccctaccac 480

gcgatcaaac cctcgcttcc gaagctggtc ggcggcacca cggcagtggt catcggagcc 540

ggtggtctcg ggcatgtcgg gatccaactg cttcgccacc tgaccccgtc ccgggtgatc 600

gctctcgacg tgagcgacga caagctcgcg ttcgcgcgcg aggtcggggc tcacgaggtg 660

gtgctctccg acgccgatgc cgtcgcgaac gtccgcaaga tcaccggcaa cgatggtgcg 720

accgccgtct tcgacttcgt cgggctgcaa cccacgctcg acatcgcgat gggcgtcgtc 780

gggaccatgg gtgacgtggt gatcgtgggc atcggtgaca tggtcgccac ggcgaaggtc 840

ggcttcttca cccagcccta cgaggtgtcg gtacgcgcgc cgtactgggg ggcgcgcgac 900

gaactcatcg aggtgctgga tctcgcacgc gatggggttc tcgaggtggc ggtcgaacga 960

ttctcactcg atgacggcgt cgaggcctac cggcgactgg ccgccaatga ccttcgaggg 1020

cgagcagtcg tggtgcctga ctga 1044

<210> 15
<211> 1020
<212> DNA
<213> Pichia trehalophila

<220>
<221> gene
<222> (1)..(1020)

<400> 15
atgtgtactt ctcaatctgg ctacgtttat cattctggta gaccactttt aactaaagaa 60

gaactttcaa ttcccgaacc aaaaggctct gaaattgttc taaaagttcg tgcagctggt 120

ttatgttcat cagatgttca tgttctaaac agcagtttac cattgactta cccaaacagt 180

tttgctatgg gtcatgaaat tgccggtgaa atttataagc ttggtccaaa cgttgacgct 240

gataaatatt caattggaga tggatatgca gttcatggtt tgaactcctg tggtgattgt 300

tccttctgta aggttggtag tcaaaacctg tgtaccgata acaattcaac ttggtacggt 360

ttaggtaaga atggtggtta tgaacagtac gttttagtta aaagtgttca tgacttaatt 420

aaaattccag aaggtgttag tttctcagag gccgcagttg cttcagatgc tgttttaact 480

ccatatcatg ctatcagcac ctgtaacttg aaggcaactt ccaaagtttt agttattggt 540

tgtggtgggt taggtacctg tgctttacaa atcatcaaat tgtacagtgc atatgttgtc 600

```
tgtgttgact ccaaagcaga attagaagaa cttgctaaag aatatggcgc tgatgaattc     660

tacaccgatt tatcaaaatc cagcgttccc aaaatgtcat ttgattgtgt ttttgatttt     720

gttgccattc agccaacttt caccatttct caaaattacg tcaagagcgg tggtatcatc     780

aaacctgttg gcttaggtgc tcctagctta acatttagtt tattggactt aggttgtaga     840

gacgttaaga tcattggctc tttctggggt acacaagctg aacaaaaaga ctgtatggaa     900

ctaattcaaa gaggtttagt caagccatta attacaagtt tcacttttga tgaatttcct     960

caagcttatg aattgttgtc gactgggaaa tccaagggta gattggttat cagtcaatag    1020


<210>  16
<211>  765
<212>  DNA
<213>  Candida nemodendra


<220>
<221>  gene
<222>  (1)..(765)

<400>  16
atgggttaca acttaatcaa caaagttgca gtcgtcacag gaggctgctc cggaattggt      60

ctcgcagtga ccaaaaaata tcttgaactg ggagcaaaag tggtcatagg agatgtatcc     120

actaaagaga agtttaacga ggtatcctcg gaactcaaag ttgcaggcct aaatgtaaac     180

aatttaaact ttgtttcagc agacagtagc aaagaagacg acaacaaacg tttagttgac     240

gaagcaatca agaactttgg tggtcttgat attgtgtgtg ctaatgccgg tatcggtagc     300

atgattccat tccatgaaat gacatttgaa gcatggagaa agttactcgc agtaaacctt     360

gatggtgtgt tcttgctaga cagattcgca attgattact ggttaaagaa tagcaaacct     420

ggtgttatcg tcaacatggg ttcaatccac tctttcgtcg ctgctccagg attagcacat     480

tactctgctt ccaagggagg tgtcaaacta ttgaccgaag ctcttgctct agagtactcg     540

tccaagggta ttagagtaaa ttctgtgaat cctgcatata ttcaaacctc attgctagaa     600

ttccttccag aagacaaaat gaatgccttg aaggcggtgc accctattgg ccgtttaggt     660

aaaccagaag aagtagccaa tgctgtcgca ttcctcagtt ccgatgaagc aaccttcata     720

catggtactt ctcttctagt tgatggaggt tacaccgctc aataa                     765


<210>  17
<211>  9
<212>  PRT
<213>  Rhodotorula mucilaginosa


<220>
<221>  PEPTIDE
<222>  (1)..(9)

<400>  17
```

Met Pro Ala Thr Leu Arg Leu Asp Lys
1               5

<210>  18
<211>  13
<212>  PRT
<213>  Rhodotorula mucilaginosa

<220>
<221>  PEPTIDE
<222>  (1)..(13)

<400>  18

Gln Ala Leu Ala Ala Pro Ser Asn Leu Ala Pro Lys Ala
1               5                   10

<210>  19
<211>  10
<212>  PRT
<213>  Rhodotorula mucilaginosa

<220>
<221>  PEPTIDE
<222>  (1)..(10)

<400>  19

Val Glu Ile Ile Lys Thr Gln Val Gln Asp
1               5                   10

<210>  20
<211>  14
<212>  PRT
<213>  Rhodotorula mucilaginosa

<220>
<221>  PEPTIDE
<222>  (1)..(14)

<400>  20

Lys Val Ala Ile Ile Thr Gly Gly Ala Ser Gly Ile Gly Leu
1               5                   10

<210>  21
<211>  8
<212>  PRT
<213>  Rhodotorula mucilaginosa

<220>
<221>  PEPTIDE
<222>  (1)..(8)

<400>  21

Ser Cys Tyr Val Thr Pro Glu Gly

1 5

<210> 22
<211> 8
<212> PRT
<213> Rhodotorula mucilaginosa

<220>
<221> PEPTIDE
<222> (1)..(8)

<400> 22

Thr Asp Phe Lys Val Asp Gly Gly
1 5

<210> 23
<211> 10
<212> PRT
<213> Rhodotorula mucilaginosa

<220>
<221> PEPTIDE
<222> (1)..(10)

<400> 23

Val Met Phe Asn Asn Ala Gly Ile Met His
1 5 10

<210> 24
<211> 10
<212> PRT
<213> Rhodotorula mucilaginosa

<220>
<221> PEPTIDE
<222> (1)..(10)

<400> 24

Val His Ala Arg Glu Gly Ile Arg Ile Asn
1 5 10

<210> 25
<211> 10
<212> PRT
<213> Pichia farinosa

<220>
<221> PEPTIDE
<222> (1)..(10)

<400> 25

Met Ala Tyr Asn Phe Thr Asn Lys Val Ala
1 5 10

<210> 26
<211> 12
<212> PRT
<213> Pichia farinosa

<220>
<221> PEPTIDE
<222> (1)..(12)

<400> 26

Thr Thr Leu Leu Val Asp Gly Gly Tyr Thr Ala Gln
1 5 10

<210> 27
<211> 9
<212> PRT
<213> Pichia farinosa

<220>
<221> PEPTIDE
<222> (1)..(9)

<400> 27

Glu Tyr Lys Glu Ala Ala Phe Thr Asn
1 5

<210> 28
<211> 16
<212> PRT
<213> Pichia farinosa

<220>
<221> PEPTIDE
<222> (1)..(16)

<400> 28

Asn Lys Val Ala Ile Ile Thr Gly Gly Ile Ser Gly Ile Gly Leu Ala
1 5 10 15

<210> 29
<211> 9
<212> PRT
<213> Pichia farinosa

<220>
<221> PEPTIDE
<222> (1)..(9)

<400> 29

Asp Val Asn Leu Asn Gly Val Phe Ser
1 5

<210>  30
<211>  9
<212>  PRT
<213>  Pichia farinosa

<220>
<221>  PEPTIDE
<222>  (1)..(9)

<400>  30

His Tyr Cys Ala Ser Lys Gly Gly Val
1               5

<210>  31
<211>  8
<212>  PRT
<213>  Pichia farinosa

<220>
<221>  PEPTIDE
<222>  (1)..(8)

<400>  31

Asn Cys Ile Asn Pro Gly Tyr Ile
1               5

<210>  32
<211>  9
<212>  PRT
<213>  Pichia farinosa

<220>
<221>  PEPTIDE
<222>  (1)..(9)

<400>  32

Leu His Pro Met Gly Arg Leu Gly Glu
1               5

<210>  33
<211>  11
<212>  PRT
<213>  Pichia stipitis

<220>
<221>  PEPTIDE
<222>  (1)..(11)

<400>  33

Met Ser Ile Pro Ala Thr Gln Tyr Gly Phe Val
1               5                   10

<210> 34
<211> 11
<212> PRT
<213> Pichia stipitis

<220>
<221> PEPTIDE
<222> (1)..(11)

<400> 34

Ser Ala Tyr Glu Gly Arg Val Val Phe Lys Pro
1 5 10

<210> 35
<211> 9
<212> PRT
<213> Pichia stipitis

<220>
<221> PEPTIDE
<222> (1)..(9)

<400> 35

Cys His Ser Asp Leu His Ala Ile Tyr
1 5

<210> 36
<211> 9
<212> PRT
<213> Pichia stipitis

<220>
<221> PEPTIDE
<222> (1)..(9)

<400> 36

Gly Tyr Gln Gln Tyr Leu Leu Val Glu
1 5

<210> 37
<211> 9
<212> PRT
<213> Pichia stipitis

<220>
<221> PEPTIDE
<222> (1)..(9)

<400> 37

Thr Phe Asp Thr Cys Gln Lys Tyr Val
1 5

<210> 38

<211> 8
<212> PRT
<213> Pichia stipitis

<220>
<221> PEPTIDE
<222> (1)..(8)

<400> 38

Leu Leu Thr Pro Tyr His Ala Met
1 5

<210> 39
<211> 9
<212> PRT
<213> Pichia stipitis

<220>
<221> PEPTIDE
<222> (1)..(9)

<400> 39

Leu Val Ser Lys Gly Lys Val Lys Pro
1 5

<210> 40
<211> 9
<212> PRT
<213> Pichia stipitis

<220>
<221> PEPTIDE
<222> (1)..(9)

<400> 40

Gly Ala Gly Gly Leu Gly Val Asn Gly
1 5

<210> 41
<211> 10
<212> PRT
<213> Pichia stipitis

<220>
<221> PEPTIDE
<222> (1)..(10)

<400> 41

Ile Gln Ile Ala Lys Ala Phe Gly Ala Thr
1 5 10

<210> 42
<211> 8

<212> PRT
<213> Pichia stipitis

<220>
<221> PEPTIDE
<222> (1)..(8)

<400> 42

Leu Gly Ser Phe Trp Gly Thr Ser
1 5

<210> 43
<211> 10
<212> PRT
<213> Leuconostoc carnosum

<220>
<221> PEPTIDE
<222> (1)..(10)

<400> 43

Met Thr Asp Arg Leu Lys Asn Lys Val Ala
1 5 10

<210> 44
<211> 12
<212> PRT
<213> Leuconostoc carnosum

<220>
<221> PEPTIDE
<222> (1)..(12)

<400> 44

Ala Glu Phe Val Val Asp Gly Gly Tyr Leu Ala Gln
1 5 10

<210> 45
<211> 9
<212> PRT
<213> Leuconostoc carnosum

<220>
<221> PEPTIDE
<222> (1)..(9)

<400> 45

Val Val Ile Thr Gly Arg Arg Ala Asn
1 5

<210> 46
<211> 9
<212> PRT

<213> Leuconostoc carnosum

<220>
<221> PEPTIDE
<222> (1)..(9)

<400> 46

Gly Gly Ala Ser Ile Ile Asn Met Ser
1 5

<210> 47
<211> 8
<212> PRT
<213> Leuconostoc carnosum

<220>
<221> PEPTIDE
<222> (1)..(8)

<400> 47

Thr Gln Thr Pro Met Gly His Ile
1 5

<210> 48
<211> 10
<212> PRT
<213> Leuconostoc carnosum

<220>
<221> PEPTIDE
<222> (1)..(10)

<400> 48

Gly Tyr Ile Lys Thr Pro Leu Val Asp Gly
1 5 10

<210> 49
<211> 11
<212> PRT
<213> Microbacterium sp.

<220>
<221> PEPTIDE
<222> (1)..(11)

<400> 49

Met Lys Ala Leu Gln Tyr Thr Lys Ile Gly Ser
1 5 10

<210> 50
<211> 14
<212> PRT
<213> Microbacterium sp.

<220>
<221> PEPTIDE
<222> (1)..(14)

<400> 50

Leu Ala Ala Gly Thr Val Arg Gly Arg Ala Val Ile Val Pro
1 5 10

<210> 51
<211> 12
<212> PRT
<213> Microbacterium sp.

<220>
<221> PEPTIDE
<222> (1)..(12)

<400> 51

Cys His Ser Asp Glu Phe Val Met Ser Leu Ser Glu
1 5 10

<210> 52
<211> 10
<212> PRT
<213> Microbacterium sp.

<220>
<221> PEPTIDE
<222> (1)..(10)

<400> 52

Val Tyr Gly Pro Trp Gly Cys Gly Arg Cys
1 5 10

<210> 53
<211> 13
<212> PRT
<213> Microbacterium sp.

<220>
<221> PEPTIDE
<222> (1)..(13)

<400> 53

Val Ser Leu Thr Asp Ala Gly Leu Thr Pro Tyr His Ala
1 5 10

<210> 54
<211> 12
<212> PRT
<213> Microbacterium sp.

<220>
<221> PEPTIDE
<222> (1)..(12)

<400> 54

Leu Arg Ala Val Ser Ala Ala Thr Val Ile Ala Leu
1 5 10

<210> 55
<211> 9
<212> PRT
<213> Microbacterium sp.

<220>
<221> PEPTIDE
<222> (1)..(9)

<400> 55

Asp Phe Val Gly Ala Asp Pro Thr Ile
1 5

<210> 56
<211> 8
<212> PRT
<213> Gordona rubropertinctus

<220>
<221> PEPTIDE
<222> (1)..(8)

<400> 56

Met Lys Ala Ile Gln Ile Ile Gln
1 5

<210> 57
<211> 10
<212> PRT
<213> Gordona rubropertinctus

<220>
<221> PEPTIDE
<222> (1)..(10)

<400> 57

Asp Leu Arg Gly Arg Ala Val Val Val Pro
1 5 10

<210> 58
<211> 9
<212> PRT
<213> Gordona rubropertinctus

<220>
<221> PEPTIDE
<222> (1)..(9)

<400> 58

Thr Ala Ala Gly Ala Cys His Ser Asp
1 5

<210> 59
<211> 11
<212> PRT
<213> Gordona rubropertinctus

<220>
<221> PEPTIDE
<222> (1)..(11)

<400> 59

Thr Pro Tyr His Ala Ile Lys Pro Ser Leu Pro
1 5 10

<210> 60
<211> 8
<212> PRT
<213> Gordona rubropertinctus

<220>
<221> PEPTIDE
<222> (1)..(8)

<400> 60

Asp Phe Val Gly Leu Gln Pro Thr
1 5

<210> 61
<211> 8
<212> PRT
<213> Gordona rubropertinctus

<220>
<221> PEPTIDE
<222> (1)..(8)

<400> 61

Val Tyr Gly Ala Trp Gly Cys Gly
1 5

<210> 62
<211> 8
<212> PRT
<213> Gordona rubropertinctus

<220>

<221>  PEPTIDE
<222>  (1)..(8)

<400>  62

Asp Asp Ala Arg His Leu Val Pro
1               5

<210>  63
<211>  8
<212>  PRT
<213>  Gordona rubropertinctus

<220>
<221>  PEPTIDE
<222>  (1)..(8)

<400>  63

Met Thr Leu Gly His Glu Gly Ala
1               5

<210>  64
<211>  14
<212>  PRT
<213>  Gordona rubropertinctus

<220>
<221>  PEPTIDE
<222>  (1)..(14)

<400>  64

Gly Gly Leu Gly His Val Gly Ile Gln Leu Leu Arg His Leu
1               5                   10

<210>  65
<211>  18
<212>  DNA
<213>  Artificial

<220>
<223>  synthetic construct

<220>
<221>  misc_feature
<222>  (1)..(18)

<400>  65
caggaaacag ctatgacc    18

<210>  66
<211>  10
<212>  PRT
<213>  Rhodotorula mucilaginosa

<220>

<221> PEPTIDE
<222> (1)..(10)

<400> 66

Val Ala Thr Ala Val Glu Thr Phe Gly Arg
1 5 10

<210> 67
<211> 9
<212> PRT
<213> Rhodotorula mucilaginosa

<220>
<221> PEPTIDE
<222> (1)..(9)

<400> 67

Phe Gly Glu Ala Val Glu Gln Ala Arg
1 5

<210> 68
<211> 20
<212> DNA
<213> Artificial

<220>
<223> synthetic construct

<220>
<221> misc_feature
<222> (1)..(20)

<400> 68
ccraaytcva cvgcvgtsgc 20

<210> 69
<211> 20
<212> DNA
<213> Artificial

<220>
<223> synthetic construct

<220>
<221> misc_feature
<222> (1)..(20)

<400> 69
gcctgytcga cvgcytcrcc 20

<210> 70
<211> 22
<212> DNA
<213> Artificial

<220>

<223> synthetic construct

<220>
<221> misc_feature
<222> (1)..(22)

<400> 70
ctccgaggtg ttgagcgcat tg 22

<210> 71
<211> 25
<212> DNA
<213> Artificial

<220>
<223> synthetic construct

<220>
<221> misc_feature
<222> (1)..(25)

<400> 71
gacgaggttc ttgatgtcgt cctcc 25

<210> 72
<211> 12
<212> PRT
<213> Pichia farinosa

<220>
<221> PEPTIDE
<222> (1)..(12)

<400> 72

Leu Leu Thr Gln Thr Leu Ala Leu Glu Gln Ala Lys
1 5 10

<210> 73
<211> 14
<212> PRT
<213> Pichia farinosa

<220>
<221> PEPTIDE
<222> (1)..(14)

<400> 73

Tyr Asn Phe Thr Asn Lys Val Ala Ile Ile Thr Gly Gly Ile
1 5 10

<210> 74
<211> 21
<212> DNA
<213> Artificial

<220>
<223> synthetic construct

<220>
<221> misc_feature
<222> (1)..(21)

<220>
<221> misc_feature
<222> (10)..(10)
<223> n is a, c, g, or t

<400> 74
ytgytcyaan gcyaadgtyt g 21

<210> 75
<211> 25
<212> DNA
<213> Artificial

<220>
<223> synthetic construct

<220>
<221> misc_feature
<222> (1)..(25)

<220>
<221> misc_feature
<222> (11)..(11)
<223> n is a, c, g, or t

<400> 75
chaayaargt ngchathaty achgg 25

<210> 76
<211> 25
<212> DNA
<213> Artificial

<220>
<223> synthetic construct

<220>
<221> misc_feature
<222> (1)..(25)

<400> 76
caacgttctg aagagatgac ttatg 25

<210> 77
<211> 20
<212> DNA
<213> Artificial

<220>
<223> synthetic construct

<220>
<221> misc_feature
<222> (1)..(20)

<400> 77
ggtggagtga agttattgac 20

<210> 78
<211> 23
<212> DNA
<213> Artificial

<220>
<223> synthetic construct

<220>
<221> misc_feature
<222> (1)..(23)

<400> 78
gccattctta gcctgttcga gag 23

<210> 79
<211> 25
<212> DNA
<213> Artificial

<220>
<223> synthetic construct

<220>
<221> misc_feature
<222> (1)..(25)

<400> 79
gtcatctctt cagaacgttg atctt 25

<210> 80
<211> 7
<212> PRT
<213> Pichia stipitis

<220>
<221> PEPTIDE
<222> (1)..(7)

<400> 80

Ala Asp Gln Val Leu Leu Lys
1 5

<210> 81
<211> 11
<212> PRT
<213> Pichia stipitis

<220>

<221> PEPTIDE
<222> (1)..(11)

<400> 81

Ile Ser Phe Asn Leu Gly Asp Leu Ala Leu Arg
1               5                   10

<210> 82
<211> 21
<212> DNA
<213> Artificial

<220>
<223> synthetic construct

<220>
<221> misc_feature
<222> (1)..(21)

<220>
<221> misc_feature
<222> (12)..(12)
<223> n is a, c, g, or t

<400> 82
gcygaycarg tnttrttraa r     21

<210> 83
<211> 21
<212> DNA
<213> Artificial

<220>
<223> synthetic construct

<220>
<221> misc_feature
<222> (1)..(21)

<400> 83
ctyaargcya artcdccyaa r     21

<210> 84
<211> 20
<212> DNA
<213> Artificial

<220>
<223> synthetic construct

<220>
<221> misc_feature
<222> (1)..(20)

<400> 84
ctaccatgcc atgagattag     20

<210> 85
<211> 20
<212> DNA
<213> Artificial

<220>
<223> synthetic construct

<220>
<221> misc_feature
<222> (1)..(20)

<400> 85
gctgtagacg tcgctaagag 20

<210> 86
<211> 20
<212> DNA
<213> Artificial

<220>
<223> synthetic construct

<220>
<221> misc_feature
<222> (1)..(20)

<400> 86
gattctcaag gctaagtcac 20

<210> 87
<211> 20
<212> DNA
<213> Artificial

<220>
<223> synthetic construct

<220>
<221> misc_feature
<222> (1)..(20)

<400> 87
gatctaacac cagctaatct 20

<210> 88
<211> 22
<212> DNA
<213> Artificial

<220>
<223> synthetic construct

<220>
<221> misc_feature
<222> (1)..(22)

<400> 88

ccaaaggagc ttatagcagt ct 22

<210> 89
<211> 29
<212> DNA
<213> Artificial

<220>
<223> synthetic construct

<220>
<221> misc_feature
<222> (1)..(29)

<400> 89
gggaaattcc atatgcctgc cactctccg 29

<210> 90
<211> 33
<212> DNA
<213> Artificial

<220>
<223> synthetic construct

<220>
<221> misc_feature
<222> (1)..(33)

<400> 90
cggcaagctt attacgcctt gggagccaag ttg 33

<210> 91
<211> 32
<212> DNA
<213> Artificial

<220>
<223> synthetic construct

<220>
<221> misc_feature
<222> (1)..(32)

<400> 91
ggaaattcca tatggcctat aacttcacta ac 32

<210> 92
<211> 33
<212> DNA
<213> Artificial

<220>
<223> synthetic construct

<220>
<221> misc_feature

<222>  (1)..(33)

<400>  92
cactgcatgc tgatggccta taacttcact aac                                   33

<210>  93
<211>  30
<212>  DNA
<213>  Artificial

<220>
<223>  synthetic construct

<220>
<221>  misc_feature
<222>  (1)..(30)

<400>  93
cgcaagctta ttactgggct gtatatcctc                                      30

<210>  94
<211>  33
<212>  DNA
<213>  Artificial

<220>
<223>  synthetic construct

<220>
<221>  misc_feature
<222>  (1)..(33)

<400>  94
ggaaattcca tatgatgtct attcctgcta cac                                   33

<210>  95
<211>  31
<212>  DNA
<213>  Artificial

<220>
<223>  synthetic construct

<220>
<221>  misc_feature
<222>  (1)..(31)

<400>  95
cactgcatgc gaatgtctat tcctgctaca c                                    31

<210>  96
<211>  33
<212>  DNA
<213>  Artificial

<220>
<223>  synthetic construct

<220>
<221> misc_feature
<222> (1)..(23)

<400> 96
cccaagctta tcatggtttg aacacgactc tac 33

<210> 97
<211> 10
<212> PRT
<213> Leuconostoc carnosum

<220>
<221> PEPTIDE
<222> (1)..(10)

<400> 97

Ile Glu Glu Thr Thr Tyr Glu Asp Trp Lys
1 5 10

<210> 98
<211> 27
<212> DNA
<213> Artificial

<220>
<223> synthetic construct

<220>
<221> misc_feature
<222> (1)..(27)

<220>
<221> misc_feature
<222> (13)..(13)
<223> n is a, c, g, or t

<400> 98
gacagawmgw ttnaarggwa argthgc 27

<210> 99
<211> 29
<212> DNA
<213> Artificial

<220>
<223> synthetic construct

<220>
<221> misc_feature
<222> (1)..(29)

<220>
<221> misc_feature
<222> (12)..(12)
<223> n is a, c, g, or t

<400> 99
gcbgtrtawc cnccrtcdac dacraaytc 29

<210> 100
<211> 23
<212> DNA
<213> Artificial

<220>
<223> synthetic construct

<220>
<221> misc_feature
<222> (1)..(23)

<400> 100
ctaagccaat accaagtgta cca 23

<210> 101
<211> 27
<212> DNA
<213> Artificial

<220>
<223> synthetic construct

<220>
<221> misc_feature
<222> (1)..(27)

<400> 101
gaacaaatcg tgctactgat tcatcac 27

<210> 102
<211> 25
<212> DNA
<213> Artificial

<220>
<223> synthetic construct

<220>
<221> misc_feature
<222> (1)..(25)

<400> 102
gaagaagccc aatcacaaag aactc 25

<210> 103
<211> 23
<212> DNA
<213> Artificial

<220>
<223> synthetic construct

<220>

<221> misc_feature
<222> (1)..(23)

<400> 103
ggcagtctat ttagctagtg aag 23

<210> 104
<211> 14
<212> PRT
<213> Microbacterium sp.

<220>
<221> PEPTIDE
<222> (1)..(14)

<400> 104

Met Lys Ala Leu Gln Tyr Thr Lys Ile Gly Ser His Pro Glu
1 5 10

<210> 105
<211> 11
<212> PRT
<213> Microbacterium sp.

<220>
<221> PEPTIDE
<222> (1)..(11)

<400> 105

Ala Tyr Glu Ala Leu Ala Ala Gly Thr Val Val
1 5 10

<210> 106
<211> 20
<212> DNA
<213> Artificial

<220>
<223> synthetic construct

<220>
<221> misc_feature
<222> (1)..(20)

<400> 106
ctscartaca cvaagatcgg 20

<210> 107
<211> 20
<212> DNA
<213> Artificial

<220>
<223> synthetic construct

<220>
<221> misc_feature
<222> (1)..(20)

<400> 107
gcbgcsagbg cytcrtabgc 20

<210> 108
<211> 20
<212> DNA
<213> Artificial

<220>
<223> synthetic construct

<220>
<221> misc_feature
<222> (1)..(20)

<400> 108
tcctcgctga ggctcatcac 20

<210> 109
<211> 22
<212> DNA
<213> Artificial

<220>
<223> synthetic construct

<220>
<221> misc_feature
<222> (1)..(22)

<400> 109
gcttctcgat ctcgacgact tc 22

<210> 110
<211> 19
<212> DNA
<213> Artificial

<220>
<223> synthetic construct

<220>
<221> misc_feature
<222> (1)..(19)

<400> 110
gcgcagcgaa ctgatcgag 19

<210> 111
<211> 22
<212> DNA
<213> Artificial

<220>

<223> synthetic construct

<220>
<221> misc_feature
<222> (1)..(22)

<400> 111
gatccagcgc tactcactcg ac 22

<210> 112
<211> 10
<212> PRT
<213> Gordona rubropertinctus

<220>
<221> PEPTIDE
<222> (1)..(10)

<400> 112

Met Lys Ala Ile Gln Ile Ile Gln Pro Gly
1 5 10

<210> 113
<211> 13
<212> PRT
<213> Gordona rubropertinctus

<220>
<221> PEPTIDE
<222> (1)..(13)

<400> 113

Val Gly Phe Phe Thr Gln Pro Tyr Glu Val Ser Val Arg
1 5 10

<210> 114
<211> 26
<212> DNA
<213> Artificial

<220>
<223> synthetic construct

<220>
<221> misc_feature
<222> (1)..(26)

<220>
<221> misc_feature
<222> (9)..(9)
<223> n is a, c, g, or t

<400> 114
atgaargcna tycaratyat ycarcc 26

<210> 115
<211> 22
<212> DNA
<213> Artificial

<220>
<223> synthetic construct

<220>
<221> misc_feature
<222> (1)..(22)

<220>
<221> misc_feature
<222> (8)..(8)
<223> n is a, c, g, or t

<220>
<221> misc_feature
<222> (14)..(14)
<223> n is a, c, g, or t

<400> 115
cytcrtangg ytgngtraar aa 22

<210> 116
<211> 21
<212> DNA
<213> Artificial

<220>
<223> synthetic construct

<220>
<221> misc_feature
<222> (1)..(21)

<400> 116
gaggacgaag tcgtccgaat g 21

<210> 117
<211> 21
<212> DNA
<213> Artificial

<220>
<223> synthetic construct

<220>
<221> misc_feature
<222> (1)..(21)

<400> 117
gccgtcacct tcagcaacac c 21

<210> 118
<211> 21
<212> DNA
<213> Artificial

<220>
<223> synthetic construct

<220>
<221> misc_feature
<222> (1)..(21)

<400> 118
ctcgacgtga gcgacgacaa g 21

<210> 119
<211> 21
<212> DNA
<213> Artificial

<220>
<223> synthetic construct

<220>
<221> misc_feature
<222> (1)..(21)

<400> 119
gcaagatcac cggcaacgat g 21

<210> 120
<211> 37
<212> DNA
<213> Artificial

<220>
<223> synthetic construct

<220>
<221> misc_feature
<222> (1)..(37)

<400> 120
catatggcta gcatgacaga tcggttaaag aataaag 37

<210> 121
<211> 35
<212> DNA
<213> Artificial

<220>
<223> synthetic construct

<220>
<221> misc_feature
<222> (1)..(35)

<400> 121
gcgcggatcc atgacagatc ggttaaagaa taaag 35

<210> 122

<211> 29
<212> DNA
<213> Artificial

<220>
<223> synthetic construct

<220>
<221> misc_feature
<222> (1)..(29)

<400> 122
cccaagcttc ttattgtgcc aaatagccg 29

<210> 123
<211> 31
<212> DNA
<213> Artificial

<220>
<223> synthetic construct

<220>
<221> misc_feature
<222> (1)..(31)

<400> 123
ggaaattcca tatgaaggca ctccagtaca c 31

<210> 124
<211> 32
<212> DNA
<213> Artificial

<220>
<223> synthetic construct

<220>
<221> misc_feature
<222> (1)..(32)

<400> 124
cactgcatgc tgatgaaggc actccagtac ac 32

<210> 125
<211> 28
<212> DNA
<213> Artificial

<220>
<223> synthetic construct

<220>
<221> misc_feature
<222> (1)..(28)

<400> 125
cccaagctta acgtcagggg acgatgac 28

<210> 126
<211> 33
<212> DNA
<213> Artificial

<220>
<223> synthetic construct

<220>
<221> misc_feature
<222> (1)..(33)

<400> 126
cactgcatgc gaatgaaggc cattcagatc atc 33

<210> 127
<211> 33
<212> DNA
<213> Artificial

<220>
<223> synthetic construct

<220>
<221> misc_feature
<222> (1)..(33)

<400> 127
cccaagctta ttagtcaggc accacgactg ctc 33

<210> 128
<211> 18
<212> DNA
<213> Artificial

<220>
<223> synthetic construct

<220>
<221> misc_feature
<222> (1)..(18)

<400> 128
tgtaaaacga cggccagt 18

<210> 129
<211> 336
<212> PRT
<213> Lodderomyces elongisporus

<400> 129

Met Ser Ile Pro Thr Thr Gln Tyr Gly Phe Val Tyr Asn Lys Ser Ser
1 5 10 15

Gly Leu Thr Leu Asn Lys Ser Ile Pro Val Ala Ser Ala Gly Val Gly

20 25 30

Gln Leu Leu Met Lys Val Asp Ser Val Gly Leu Cys His Ser Asp Leu
35 40 45

His Val Ile Tyr Glu Gly Leu Asp Cys Gly Asp Asn Tyr Val Met Gly
50 55 60

His Glu Ile Ala Gly Thr Val Val Asp Val Gly Pro Glu Val Asp Arg
65 70 75 80

Trp Asn Val Gly Asp Arg Val Ala Ala Val Gly Pro Asn Gly Cys Gly
85 90 95

Gly Cys Arg Ala Cys Arg Asp Gly Ile Glu Asn Val Cys Lys His Ser
100 105 110

Phe Gly Asn Trp Tyr Gly Leu Gly Ser Asp Gly Gly Tyr Gln Gln Tyr
115 120 125

Leu Leu Val Gln Lys Pro Arg Asn Leu Val Lys Ile Pro Asp Asn Val
130 135 140

Pro Ser Asp Val Ala Ala Ala Ser Thr Asp Ala Val Leu Thr Pro Tyr
145 150 155 160

His Ala Ile Lys Met Ala Gly Val Gly Pro Thr Ser Lys Val Leu Ile
165 170 175

Val Gly Ala Gly Gly Leu Gly Cys Asn Ala Val Gln Val Ala Lys Ala
180 185 190

Phe Gly Ala His Val Thr Ile Leu Asp Lys Lys Glu Arg Ala Arg Ala
195 200 205

Glu Ala Val Lys Phe Gly Ala Asp Val Ala Tyr Glu Ser Leu Pro Leu
210 215 220

Ser Thr Glu Pro Gly Ser Phe Asp Ala Cys Leu Asp Phe Val Ser Val
225 230 235 240

Gln Ala Thr Phe Gly Ile Cys Gln Lys Phe Cys Ala Pro Lys Gly Cys
245 250 255

Ile Ile Pro Ala Gly Leu Gly Ala Pro Lys Leu Thr Leu Asp Leu Ala
260 265 270

Asp Leu Asp Leu Arg Glu Ile Arg Ile Leu Gly Thr Phe Trp Gly Thr
275 280 285

```
Ala Thr Asp Leu Glu Glu Val Phe Asp Leu Val Gly Lys Gly Leu Val
    290                 295                 300

Lys Pro Met Val Arg Ala Ala Lys Leu Glu Glu Leu Pro Asp Tyr Ile
305                 310                 315                 320

Glu Lys Leu Arg Lys Asn Glu Tyr Glu Gly Arg Ile Val Phe Asn Pro
                325                 330                 335


<210>  130
<211>  1012
<212>  DNA
<213>  Lodderomyces elongisporus


<220>
<221>  gene
<222>  (1)..(1011)

<400>  130
atgtcaattc caactaccca atatggtttt gtttacaata agtcgtctgg cttaacattg      60

aacaagagta tacctgttgc ctcggcaggt gtgggtcaat tgcttatgaa ggttgactct     120

gttggattgt gccactcgga cctccatgtg atttacgaag gtttggattg tggtgataac     180

tatgtcatgg gccatgagat tgccggtacc gtggttgatg ttggtccaga ggttgataga     240

tggaatgttg gtgatagagt tgccgctgtg ggtccaaatg gttgtggtgg ttgcagagcc     300

tgtcgcgacg gaattgaaaa tgtatgtaaa cactcttttg gtaattggta tggcttgggc     360

tcagatggcg gataccaaca atatttgctt gtgcaaaaac cacgcaattt ggttaagatt     420

cctgacaatg ttccttcgga tgtagctgca gcctcgactg acgctgtatt gacaccatac     480

cacgcaatca agatggctgg tgtggggcca acatcaaagg tgctcattgt tggcgctggt     540

ggcttgggct gcaatgccgt gcaagttgcc aaggcatttg gtgctcatgt cactattttg     600

gacaagaagg aacgcgcgcg cgctgaagct gtcaagtttg gtgccgacgt tgcctatgag     660

agcttaccac tgagcaccga gccaggctca tttgatgcat gtttggattt tgtttctgtg     720

caagcaacgt ttggcatttg ccaaaagttt tgtgcaccaa aaggttgcat catccccgcg     780

gggctcggtg caccaaagtt gacgcttgat ttggcagatt tggatttgcg cgaaattcgt     840

attttgggta ctttttgggg aaccgcgacc gatttggagg aggtgtttga cttggttgga     900

aagggacttg ttaagcccat ggtgcgtgca gccaagttgg aggaattgcc agactatatt     960

gaaaagttga gaaagaatga atatgaaggt agaattgtct ttaatccata ag            1012
```

## Beschreibung

Die vorliegende Erfindung betrifft ein Verfahren zur enantioselektiven enzymatischen Reduktion einer Ketoverbindung zur entsprechenden chiralen Hydroxyverbindung, wobei die Ketoverbindung mit einer Oxidoreduktase reduziert wird. Die Erfindung betrifft ferner neue Oxidoreduktasen zur Verwendung bei der enantioselektiven Reduktion von Ketoverbindungen zu chiralen Hydroxyverbindungen.

Optisch aktive Hydroxyverbindungen sind wertvolle chirale Bausteine mit breiter Anwendung für die Synthese von pharmakologisch wirksamen Verbindungen, aromatischen Substanzen, Pheromonen, Agrochemikalien und Enzyminhibitoren. Dabei ist insbesondere im Pharmasektor ein steigender Bedarf an chiralen Verbindungen und somit chiralen Synthesetechnologien zu verzeichnen, da racemische Verbindungen in Zukunft kaum noch als Arzneimittel Verwendung finden werden.

Die asymmetrische Reduktion prochiraler Ketoverbindungen ist ein Sektor der stereoselektiven Katalyse, in dem die Biokatalyse eine leistungsfähige Konkurrenztechnologie zur chemischen Katalyse darstellt. Die chemische asymmetrische Hydrierung erfordert den Einsatz hochgiftiger und umweltbelastender Schwermetallkatalysatoren, extremer und somit energieintensiver Reaktionsbedingungen sowie große Mengen organischer Lösungsmittel. Des weiteren sind diese Verfahren häufig gekennzeichnet durch Nebenreaktionen und unzureichende Enantiomerenüberschüsse.

Reduktionen prochiraler Ketoverbindungen zu Hydroxyverbindungen und umgekehrt kommen in der Natur in zahlreichen biochemischen Pathways, sowohl im Primärstoffwechsel als auch im Sekundärstoffwechsel, in jedem Organismus vor und werden von unterschiedlichen Typen von sekundären Alkoholdehydrogenasen und Oxidoreduktasen katalysiert. Diese Enzyme sind in der Regel cofaktorabhängig.

Die prinzipielle Machbarkeit der Nutzung von Biokatalysatoren zu Reduktion von prochiralen Ketoverbindungen zu chiralen Hydroxyverbindungen wurde in der Vergangenheit wiederholt anhand von Modellsystemen demonstriert, wobei sowohl mit isolierten Oxidoreduktasen als auch mit unterschiedlichen Ganzzellbiotransformationssystemen gearbeitet wurde. Dabei erwies sich der biokatalytische Ansatz im wesentlichen hinsichtlich der milden Reaktionsbedingungen, fehlender Nebenprodukte und oft wesentlich besseren erreichbaren Enantiomerenüberschüssen als vorteilhaft. Die Verwendung isolierter Enzyme ist gegenüber Verfahren mit ganzen Zellen hinsichtlich des erreichbaren Enantiomerenüberschuss, der Entstehung von Abbau- und Nebenprodukten als auch hinsichtlich der Produktisolation von Vorteil. Des weiteren ist die Verwendung von Ganzzellprozessen nicht jedem Chemieunternehmen möglich, da dafür spezielle Ausrüstung und Know-How erforderlich sind.

Jüngst konnte gezeigt werden, dass die Verwendung isolierter Oxidoreduktasen in wässrig/organischen Zwei -Phasensystemen mit organischen Lösungsmitteln äußerst effizient, ökonomisch und auch in hohen Konzentrationen (> 5%) möglich ist. Bei den beschriebenen Systemen bildet dabei die zu reduzierende, meist schlecht wasserlösliche Ketoverbindung zusammen mit dem organischen Lösungsmittel die organische Phase. Teilweise kann auch auf das organische Lösungsmittel selbst verzichtet werden, dann wird die organische Phase von der zu reduzierenden Ketoverbindung gebildet (DE 10119274, DE 10327454.4, DE 103 37 401.9, DE 103 00 335.5). Die Coenzymregenerierung wird dabei durch die gleichzeitige Oxidation sekundärer Alkohole realisiert, wobei in den meisten Fällen das preiswerte wassermischbare 2-Propanol verwendet wird.

Beispiele für geeignete R-und S-spezifische Oxidoreduktasen und Dehydrogenasen hoher Enantioselektivität sind:

*Carbonylreduktase aus Candida parapsilosis* (CPCR) (US 5,523,223 und US 5,763,236, (Enzyme Microb Technol. 1993 Nov; 15(11):950-8)) und *Pichia capsulata* (DE10327454.4). *Carbonylreduktase aus Rhodococcus erythropolis* (RECR) (US 5,523,223), *Norcardia fusca* (Biosci. Biotechnol. Biochem.,63 (10) (1999), Seiten 1721-1729), (Appl Microbiol Biotechnol. 2003 Sep;62(4):380-6. Epub 2003 Apr 26), und *Rhodococcus ruber* (J Org Chem. 2003 Jan 24;68(2):402-6.).

R-spezifische sekundären Alkoholdehydrogenasen aus Organismen der Gattung *Lactobacillus* (Lactobacillus kefir (US5200335), Lactobacillus brevis (DE 19610984 A1) (Acta Crystallogr D Biol Crystallogr. 2000 Dec;56 Pt 12: 1696-8), Lactobacillus minor (DE10119274) oder *Pseudomonas* (US 05385833)(Appl Microbiol Biotechnol. 2002 Aug; 59(4-5):483-7. Epub 2002 Jun 26.,J. Org. Chem. 1992, 57, 1532)

Die heute bekannten Enzyme reichen aber bei weitem nicht aus, um das gesamte Marktpotential an stereoselektiven Reduktionen von Ketoverbindungen auszuschöpfen. Das ist einerseits damit zu begründen, dass die einzelnen Enzyme über sehr unterschiedliche Eigenschaften bezüglich Substratspektrum, pH-Optima sowie Temperatur- und Lösungsmittelstabilitäten verfügen, die sich häufig gegenseitig ergänzen. Daher können selbst verhältnismäßig ähnliche, homologe Enzyme im Hinblick auf ein bestimmtes Substrat ein völlig unterschiedliches Umsetzungsverhalten aufweisen. Andererseits sind längst nicht alle beschriebenen Enzyme kloniert und in ausreichendem Maße überexprimierbar, was bedeutet, daß diese Enzyme für eine industrielle Anwendung nicht zur Verfügung stehen. Daher ist es zur möglichst breiten Ausschöpfung des synthetischen Potentials der enzymatischen asymmetrischen Hydrierung notwendig, über ein möglichst breites Portfolio industriell zugänglicher unterschiedlicher Oxidoreduktasen zu verfügen, die zudem für eine Anwendung in wässerig/organischen Zweiphasensystemen mit organischen Lösungsmitteln geeignet sind.

Gegenstand der vorliegenden Erfindung sind nun eine Reihe neuer, enantioselektiver, R-und S-spezifischer Oxidoreduktasen, die sich durch gute Stabilität in wässerig/organischen Zweiphasensystemen sowie durch gute Exprimierbarkeit in Escherichia coi (>500 Units/g E. coli Biofeuchtmasse) auszeichnen, sowie ein Verfahren zur enantioselektiven enzymatischen Reduktion einer Ketoverbindung zur entsprechenden chiralen Hydroxyverbindung.

Die erfindungsgemäßen Oxidoreduktasen sind dadurch gekennzeichnet, daß sie eine Aminosäuresequenz aufweisen, bei welcher

(a) mindestens 70% der Aminosäuren identisch sind mit den Aminosäuren einer der Aminosäuresequenzen SEQ ID No 1, SEQ ID No 6 und SEQ ID No 8, oder
(b) mindestens 55% der Aminosäuren identisch sind mit den Aminosäuren der Aminosäuresequenz SEQ ID No 2, oder
(c) mindestens 65% der Aminosäuren identisch sind mit den Aminosäuren der Aminosäuresequenz SEQ ID No 3, oder
(d) mindestens 75% der Aminosäuren identisch sind mit den Aminosäuren der Aminosäuresequenz SEQ ID No 4, oder
(e) mindestens 65% der Aminosäuren identisch sind mit den Aminosäuren der Aminosäuresequenz SEQ ID No 5, oder
(f) mindestens 50 % der Aminosäuren identisch sind mit den Aminosäuren der Aminosäuresequenz SEQ ID No 7.
(g) mindestens 72 % der Aminosäuren identisch sind mit den Aminosäuren der Aminosäuresequenz SEQ ID No 129.

Das Polypeptid entsprechend SEQ ID No 1 kann aus Hefen, insbesondere aus Hefen der Gattung Rhodotorula, insbesondere aus Rhodotorula mucilaginosa, gewonnen werden. Gegenstand der Erfindung ist weiterhin eine Nukleinsäuresequenz SEQ ID No 9, die für das Protein mit der Aminosäuresequenz SEQ ID No 1 codiert.

Die Oxidoreduktase aus Rhodotorula mucilaginosa reduziert beispielsweise 2-Oktanon zu S-2-Oktanol und oxidiert von beiden Enantiomeren des 2-Oktanol bevorzugt S-2-Oktanol. Die Oxidoreduktase aus Rhodotorula mucilaginosa ist beispielsweise ein Homodimer mit einem Molekulargewicht bestimmt im SDS - Gel von 30± 2 k Da. Das pH -Optimum für die Reduktionsreaktion liegt für diese Oxidoreduktase im Bereich von 7,0 bis 8,0 und das pH - Optimum für die Oxidationsreaktion liegt im Bereich von 8,5-10. Die Oxidoreduktase aus Rhodotorula mucilaginosa weist eine gute Temperatur - und pH - Stabilität auf und zeigt im pH-Bereich von 5,5 bis 10 und bei Temperaturen bis 35˚C auch bei Inkubationszeiten von mehreren Stunden nur geringen Aktivitätsverlust. Ferner weist die Oxidoreduktase aus Rhodotorula mucilaginosa eine hohe Stabilität in organischen Lösungsmitteln auf. Polypeptide entsprechend SEQ ID No 2 oder SEQ ID No 8 können aus Hefen, insbesondere aus Hefen der Gattungen *Pichia, Candida, Pachysolen, Debaromyces* oder *Issatschenkia,* insbesondere aus Pichia farinosa DSMZ 3316 oder Candida nemodendra DSMZ 70647, gewonnen werden. Gegenstand der Erfindung ist weiterhin eine Nukleinsäuresequenz SEQ ID No 10 und eine Nukleinsäuresequenz SEQ ID No 16, die für die Aminosäuresequenzen SEQ ID No 2 und SEQ ID No 8 codieren. Die Oxidoreduktase reduziert 2-Butanon bevorzugt zu R-2-Butanol und oxydiert von beiden Enantiomeren des 2-Butanol bevorzugt R-2-Butanol. Die Oxidoreduktase aus Pichia farinosa weist dabei eine wesentlich höhere Aktivität gegenüber R-2-Butanol und 2-Propanol auf als gegenüber R-2-Oktanol, des weiteren weist das Enzym eine wesentlich höhere Aktivität gegenüber Aceton und 2-Butanon auf als gegenüber 2-Oktanon.

Die Oxidoreduktase aus Candida nemodendra weist hingegen eine ähnliche Aktivität gegenüber R-2-Butanol, 2-Propanol und R-2-Oktanol auf, des weiteren weist das Enzym auch eine in etwa ähnliche Aktivität gegenüber 2-Oktanon auf.

Die Oxidoreduktase aus Pichia farinosa ist ein Homodimer mit einem Molekulargewicht bestimmt im SDS-Gel von 27± 2 k Da. Das pH-Optimum für die Reduktionsreaktion liegt für diese Oxidoreduktase im Bereich von 5,0 bis 6,0 und das pH-Optimum für die Oxidationsreaktion liegt im Bereich von 7,5-10. Die Oxidoreduktase aus Pichia farinosa weist eine gute pH- und Lösungsmittelstabilität auf und zeigt im pH-Bereich von 5,5 bis 10 auch bei Inkubationszeiten von mehreren Stunden nur geringen Aktivitätsverlust.

Die Oxidoreduktase aus Candida nemodendra ist ein Homomer mit einem Molekulargewicht bestimmt im SDS - Gel von 27± 2 k Da. Das pH-Optimum für die Reduktionsreaktion liegt für diese Oxidoreduktase bei 6 und das pH -Optimum für die Oxidationsreaktion liegt im Bereich von 10-11. Die Oxidoreduktase aus Candida nemodendra weist eine gute pH- und Lösungsmittelstabilität auf und zeigt im pH-Bereich von 6,5 bis 9,5 auch bei Inkubationszeiten von mehreren Stunden nur geringen Aktivitätsverlust.

Die Polypeptide entsprechend SEQ ID No 3 oder SEQ ID No 7 können aus Hefen, insbesondere aus Hefen der Gattungen *Pichia* und *Candida,* insbesondere aus Pichia stipidis DSMZ 3651 und Pichia trehalophila DSMZ 70391, gewonnen werden. Gegenstand der Erfindung ist weiterhin eine Nukleinsäuresequenz SEQ ID No 11 und eine Nukleinsäuresequenz SEQ ID No 15, die für die Polypeptide SEQ ID No 3 bzw. SEQ ID No 7 codieren.

Die Carbonylreduktase aus Hefen der Gattung Pichia und Candida, die mindestens 65% Identität zu der Aminosäuresequenz SEQ ID No 3 oder mindestens 50%-Identität zu der Aminosäuresequenz SEQ ID No 7 aufweisen, reduzieren 2-Oktanon bevorzugt zu S-2-Oktanol und oxydieren von beiden Enantiomeren des 2-Oktanol bevorzugt S-2-Oktanol. Sie eignen sich auch besonders zur Reduktion von 4-Haloacetoacetat ester zu R-4-Halo-3-hydroxybuttersäureestern.

Die Oxidoreduktase aus Pichia stipidis ist ein Homodimer mit einem Molekulargewicht bestimmt im SDS-Gel von 36± 2 k Da. Das pH-Optimum für die Reduktionsreaktion liegt für diese Oxidoreduktase im Bereich von 5,5 bis 6,5 und das pH-Optimum für die Oxidationsreaktion liegt im Bereich von 6,5-8,0. Die Oxidoreduktase aus Pichia stipidis weist eine gute pH- und Lösungsmittelstabilität auf und zeigt im pH-Bereich von 5,5 bis 10 auch bei Inkubationszeiten von mehreren Stunden nur geringen Aktivitätsverlust.

Die Oxidoreduktase aus Pichia trehalophila ist Homomer mit einem Molekulargewicht bestimmt im SDS-Gel von 36± 2 k Da. Das pH-Optimum für die Reduktionsreaktion liegt für diese Oxidoreduktase im Bereich von 7-7,5 und das pH-Optimum für die Oxidationsreaktion liegt im Bereich von 7-8 .

Das Polypeptid entsprechend SEQ ID No 4 kann aus Bakterien der Gruppe Leuconostoc, insbesondere aus Leuconostoc carnosum DSMZ 5576 gewonnen werden. Gegenstand der Erfindung ist weiterhin eine Nukleinsäuresequenz SEQ ID No 12, welche für ein Protein mit der Aminosäuresequenz SEQ ID No 4 codiert. Das Polypeptid eignet sich besonders zur Reduktion von 2-Oktanon zu R-2-Oktanol und zur Oxydation von R-2-Oktanol. Es eignet sich auch sehr gut zur Reduktion von 4-Haloacetoacetat ester zu S-4-Halo-3-hydroxybuttersäureestern.

Die Oxidoreduktase aus Leuconostoc carnosum ist ein Homodimer mit einem Molekulargewicht bestimmt im SDS - Gel von 27± 2 k Da. Das pH -Optimum für die Reduktionsreaktion liegt für diese Oxidoreduktase im Bereich von 5,0 bis 6,0 und das pH - Optimum für die Oxidationsreaktion liegt im Bereich von 6,0-9,0. Die Oxidoreduktase aus Leuconostoc carnosum weist eine gute Temperatur, pH - und Lösungsmittelstabilität auf und zeigt im pH-Bereich von 4,5 bis 10 sowie bei Temperaturen bis 35˚C auch bei Inkubationszeiten von mehreren Stunden nur geringen Aktivitätsverlust.

Das Polypeptid entsprechend SEQ ID No 5 kann aus Bakterien der Gruppe Actinobacteria , insbesondere aus Bakterien der Gruppe Microbacterium, insbesondere aus Microbacterium spec. DSMZ 20028 gewonnen werden. Gegenstand der Erfindung ist weiterhin eine Nukleinsäuresequenz SEQ ID No 13, die für das Protein mit der Aminosäuresequenz SEQ ID No 5 codiert. Das Polypeptid eignet sich sehr gut zur Reduktion von 2-Octanone zu S-2-Oktanol und oxydiert von beiden Enantiomeren des 2-Oktanol bevorzugt S-2-Oktanol oxidiert. Es eignet sich auch sehr gut zur Reduktion von 4-Haloacetoacetat ester zu R-4-Halo-3-hydroxybuttersäureestern.

Die Oxidoreduktase aus Microbacterium spec. DSMZ 20028 ist beispielsweise ein Homotetramer mit einem Molekulargewicht bestimmt im SDS - Gel von 35 ± 2 k Da. Das pH -Optimum für die Reduktionsreaktion liegt für diese Oxidoreduktase im Bereich von 6,0 bis 7,5 und das pH -Optimum für die Oxidationsreaktion liegt im Bereich von 7,5-9,5. Die Oxidoreduktase aus Microbacterium spec weist eine gute Temperatur, pH - und Lösungsmittelstabilität auf und zeigt im pH-Bereich von 4,5 bis 10 sowie bei Temperaturen bis 50˚C auch bei Inkubationszeiten von mehreren Stunden nur geringen Aktivitätsverlust.

Das Polypeptid entsprechend SEQ ID No 6 kann aus Bakterien der Gruppe Actinobacteria , insbesondere aus Bakterien der Gruppe Gordonia, insbesondere aus Gordonia rubripertincta DSMZ 43570 gewonnen werden. Gegenstand der Erfindung ist weiterhin eine Nukleinsäuresequenz SEQ ID No 14, die für das Protein mit der Aminosäuresequenz SEQ ID No 6 codiert. Das Polypeptid eignet sich sehr gut zur Reduktion von 2-Octanone zu S-2-Oktanol und oxydiert von beiden Enantiomeren des 2-Oktanol bevorzugt S-2-Oktanol oxidiert. Es eignet sich auch sehr gut zur Reduktion von 4-Haloacetoacetat ester zu R-4-Halo-3-hydroxybuttersäureestern.

Die Oxidoreduktase aus Gordonia rubripertincta DSMZ 43570 ist ein Homomer mit einem Molekulargewicht bestimmt im SDS-Gel von 41 ± 3 k Da. Das pH-Optimum für die Reduktionsreaktion liegt für diese Oxidoreduktase im Bereich von 4,5 bis 5,5 und das pH-Optimum für die Oxidationsreaktion liegt im Bereich von 7,5 bis 9,5. Die Oxidoreduktase aus Gordonia rubripertincta DSMZ 43570 weist eine gute Temperatur, pH- und Lösungsmittelstabilität auf und zeigt im pH-Bereich von 4,5-10 sowie bei Temperaturen bis 55 ˚C auch bei Inkubationszeiten von mehreren Stunden nur geringen Aktivitätsverlust.

Das Polypeptid entsprechend SEQ ID No 129 kann aus Hefen, insbesondere aus Hefen der Gattungen Lodderomyces, insbesondere aus Lodderomyces elongisporus DSMZ 70320 gewonnen werden. Gegenstand der Erfindung ist weiterhin eine Nukleinsäuresequenz SEQ ID No 130, die für das Protein mit der Aminosäuresequenz SEQ ID No 129 codiert. Das Polypeptid eignet sich sehr gut zur Reduktion von 2-Octanone zu S-2-Oktanol und oxydiert von beiden Enantiomeren des 2-Oktanol bevorzugt S-2-Oktanol oxidiert. Es eignet sich auch sehr gut zur Reduktion von 4-Haloacetoacetat ester zu R-4-Halo-3-hydroxybuttersäureestern. Die Erfindung betrifft weiterhin Fusionsproteine, die dadurch gekennzeichnet sind, dass sie Oxidoreduktasen mit den Aminosäuresequenzen SEQ ID No 1, SEQ ID No 2, SEQ ID No 3, SEQ ID No 4, SEQ ID No 5, SEQ ID No 6, SEQ ID No 7, SEQ ID No 8 , SEQ ID No 129 oder deren Homologen darstellen, die mit einem weiteren Polypeptid am N-Terminalen oder Carboxy-terminalen Ende peptidisch verbunden sind. Fusionsproteine können beispielsweise leichter von anderen Proteinen abgetrennt werden oder können in größeren Mengen rekombinant exprimiert werden.

Die Erfindung betrifft ferner Antikörper, die spezifisch an die Oxidoreduktasen gemäß SEQ ID No 1, SEQ ID No 2, SEQ ID No 3, SEQ ID No 4, SEQ ID No 5, SEQ ID No 6, SEQ ID No 7, SEQ ID No 8 , SEQ ID No 129 oder deren Homologen binden. Die Herstellung dieser Antikörper erfolgt nach bekannten Methoden durch Immunisierung von geeigneten Säugetieren und anschließender Gewinnung der Antikörper. Die Antikörper können monoklonal oder polyklonal sein.

Vergleiche von Aminosäuresequenzen können beispielsweise im Internet in Proteindatenbaken wie z.B. SWISS-PROT, PIR sowie in DNA-Datenbanken wie z.B.. EMBL, GenBank etc unter Nutzung des FASTA-Programm oder BLAST-Programm durchgeführt werden.

Das optimale Alignement wird dabei mit Hilfe des BLAST -Algorithmus (Basic Local Alignement Search Tool) ermittelt (Altschul et al. 1990, Proc. Natl. Acd. Sci. USA. 87: 2264-2268). Als Scoring-Matrix zur Berechnung der Sequenzähnlichkeit wird die PAM30-Matrix zugrunde gelegt. (Dayhoff; M.O., Schwarz, R.M., Orcutt, B.C. 1978. "A model of evolutionary change in Proteins" in "Atlas of Protein Sequence and structure" 5(3) M.O. Dayhoff (ed) 345-352, National Biomedical Research foundation*).*

Die Erfindung betrifft ferner Proteinfragmente, die dadurch gekennzeichnet sind, dass sie Fragmente der Aminosäuresequenz SEQ ID No 1 darstellen, mit einer Anzahl von mehr als 26 Aminosäuren je Fragment.

Gegenstand der Erfindung ist weiterhin eine mikrobielle Carbonyldehydrogenase die N-terminal die Aminosäuresequenz MPATLRLDK (SEQ ID No 17) und/oder C-terminal die Aminosäuresequenz QALAAPSNLAPKA (SEQ ID No 18) und oder eine der internen Partialsequenzen VEIIKTQVQD (SEQ ID No 19), KVAIITGGASGIGL (SEQ ID No 20), SCYVTPEG (SEQ ID No 21), TDFKVDGG (SEQ ID No 22), VMFNNAGIMH (SEQ ID No 23), oder VHAREGIRIN (SEQ ID No 24) enthält.

Die Erfindung betrifft ferner Proteinfragmente, die dadurch gekennzeichnet sind, dass sie Fragmente der Aminosäuresequenz SEQ ID No 2 darstellen, mit einer Anzahl von mehr als 15 Aminosäuren je Fragment.

Gegenstand der Erfindung ist weiterhin eine mikrobielle Carbonyldehydrogenase die N-terminal die Aminosäuresequenz MAYNFTNKVA (SEQ ID No 25) und/oder C-terminal die Aminosäuresequenz TTLLVDGGYTAQ (SEQ ID No 26) und oder eine der internen Partialsequenzen EYKEAAFTN (SEQ ID No 27), NKVAIITGGISGIGLA (SEQ ID No 28), DVNLNGVFS (SEQ ID No 29), HYCASKGGV (SEQ ID No 30), NCINPGYI (SEQ ID No 31), oder LHPMGRLGE (SEQ ID No 32) enthält.

Die Erfindung betrifft ferner Proteinfragmente, die dadurch gekennzeichnet sind, dass sie Fragmente der Aminosäuresequenz SEQ ID No 3 darstellen, mit einer Anzahl von mehr als 15 Aminosäuren je Fragment.

Gegenstand der Erfindung ist weiterhin eine mikrobielle Carbonyldehydrogenase die N-terminal die Aminosäuresequenz MSIPATQYGFV (SEQ ID No 33) und/oder C-terminal die Aminosäuresequenz SAYEGRVVFKP (SEQ ID No 34) und/ oder eine der internen Partialsequenzen CHSDLHAIY (SEQ ID No 35), GYQQYLLVE (SEQ ID No 36), TFDTCQKYV (SEQ ID No 37), LLTPYHAM (SEQ ID No 38), LVSKGKVKP (SEQ ID No 39), GAGGLGVNG (SEQ ID No 40), IQIAKAFGAT (SEQ ID No 41) oder LGSFWGTS (SEQ ID No 42) enthält.

Die Erfindung betrifft ferner Proteinfragmente, die dadurch gekennzeichnet sind, dass sie Fragmente der Aminosäuresequenz SEQ ID No 4 darstellen, mit einer Anzahl von mehr als 18 Aminosäuren je Fragment.

Gegenstand der Erfindung ist weiterhin eine mikrobielle Carbonyldehydrogenase die N-terminal die Aminosäuresequenz MTDRLKNKVA (SEQ ID No 43) und/oder C-terminal die Aminosäuresequenz AEFVVDGGYLAQ (SEQ ID No 44) und/ oder eine der internen Partialsequenzen VVITGRRAN (SEQ ID No 45), GGASIINMS (SEQ ID No 46), TQTPMGHI (SEQ ID No 47) oder GYIKTPLVDG (SEQ ID No 48) enthält.

Die Erfindung betrifft des weiteren Proteinfragmente, die dadurch gekennzeichnet sind, dass sie Fragmente der Aminosäuresequenz SEQ ID No 5 darstellen, mit einer Anzahl von mehr als 18 Aminosäuren je Fragment.

Gegenstand der Erfindung ist weiterhin eine mikrobielle Carbonyldehydrogenase die N-terminal die Aminosäuresequenz MKALQYTKIGS (SEQ ID No 49) und/oder C-terminal die Aminosäuresequenz LAAGTVRGRAVIVP (SEQ ID No 50) und/ oder eine der internen Partialsequenzen CHSDEFVMSLSE (SEQ ID No 51), VYGPWGCGRC (SEQ ID No 52), VSLTDAGLTPYHA (SEQ ID No 53), LRAVSAATVIAL (SEQ ID No 54) oder DFVGADPTI (SEQ ID No 55) enthält.

Die Erfindung betrifft ebenso Proteinfragmente, die dadurch gekennzeichnet sind, dass sie Fragmente der Aminosäuresequenz SEQ ID No 6 darstellen, mit einer Anzahl von mehr als 26 Aminosäuren je Fragment.

Gegenstand der Erfindung ist weiterhin eine mikrobielle Carbonyldehydrogenase die N-terminal die Aminosäuresequenz MKAIQIIQ (SEQ ID No 56) und/oder C-terminal die Aminosäuresequenz DLRGRAVVVP (SEQ ID No 57) und/ oder eine der internen Partialsequenzen TAAGACHSD (SEQ ID No 58), TPYHAIKPSLP(SEQ ID No 59), DFVGLQPT (SEQ ID No 60), VYGAWGCG (SEQ ID No 61), DDARHLVP (SEQ ID No 62), MTLGHEGA (SEQ ID No 63) oder GGLGHVGIQLLRHL (SEQ ID No 64) enthält.

Die Erfindung betrifft ferner einen Klonierungsvektor, enthaltend eine oder mehrere Nukleinsäuresequenzen codierend für die Carbonylreduktasen gemäß SEQ ID No 1, SEQ ID No 2, SEQ ID No 3, SEQ ID No 4, SEQ ID No 5, SEQ ID No 6, SEQ ID No 7, SEQ ID No 8, SEQ ID No 129 oder deren Homologen. Die Erfindung umfasst des weiteren einen solchen Klonierungsvektor der zusätzlich zur Carbonylreduktase ein geeignetes Enzym zur Regenerierung des NAD(P) enthält, wie z.B. Formatdehydrogenasen, Alkoholdehydrogenasen oder Glucosedehydrogenase.

Die Erfindung betrifft weiterhin einen Expressionsvektor, der sich in einer Bakterien., Insekten-, Pflanzen- oder Säugetierzelle befindet und eine Nukleinsäuresequenz enthält die für die Carbonylreduktasen gemäß SEQ ID No 1, SEQ ID No 2, SEQ ID No 3, SEQ ID No 4, SEQ ID No 5, SEQ ID No 6, SEQ ID No 7, SEQ ID No 8, SEQ ID No 129 oder deren Homologen kodiert und die in geeigneter Weise mit einer Expressionskontrollsequenz verbunden ist. Die Erfindung betrifft ferner eine rekombinante Wirtszelle, die eine Bakterien-, Hefe, Insekten-, Pflanzen-, oder Säugetierzelle ist und mit einem solchen Expressionsvektor transformiert oder transfektiert wurde sowie ein Herstellungsverfahren zur Gewinnung einer Carbonylreduktase die auf Kultivierung einer solchen rekombinanten Wirtszelle beruht.

Geeignete Klonierungsvektoren sind beispielsweise ppCR-Script, pCMV-Script , pBluescript (Stratagene), pDrive cloning Vector (Quiagen, Hilden, Deutschland), pS Blue, pET Blue, pET LIC-Vektoren (Novagen, Madison, USA) sowie TA-PCR Klonierungsvektoren (Invitrogen, Karlsruhe, Deutschland).

Geeignete Expressionsvektoren sind beispielsweise pKK223-3, pTrc99a, pUC, pTZ, pSK, pBluescript, pGEM, pQE, pET, PHUB, pPLc, pKC30, pRM1/pRM9, pTrxFus,pAS1, pGEx, pMAL oder pTrx.

Geeignete Expressionskontrollsequenzen sind beispielsweise trp-lac (tac)-Promotor, trp-lac (trc) promotor, lac-Promotor, T7-Promotor oder λpL-Promotor.

Die Oxidoreduktasen gemäß SEQ ID No 1, SEQ ID No 2, SEQ ID No 3, SEQ ID No 4, SEQ ID No 5, SEQ ID No 6, SEQ ID No 7, SEQ ID No 8, SEQ ID No 129 oder deren Homologen lassen sich so gewinnen, dass die oben genannten rekombinanten E. coli Zellen kultiviert werden, die Expression der entsprechenden Oxidoreduktase induziert wird und anschließend nach etwa 10 bis 18 Stunden (h) die Zellen durch Ultraschallbehandlung, durch Naßvermahlung mit Glasperlen in einer Kugelmühle (Retsch, GmbH, Haan Deutschland 10 min, 24 Hz) oder mittels Hochdruckhomogenisator aufgeschlossen werden. Der erhaltene Zellextrakt kann entweder direkt verwendet werden oder weiter gereinigt werden. Dazu wird der Zellextrakt beispielsweise zentrifugiert und der erhaltene Überstand wird einer Ionenaustauschchromatographie unterworfen, beispielsweise durch Ionenaustauschchromatographie am Q-Sepharose Fast Flow ® (Pharmacia).

Die Erfindung betrifft ferner ein Verfahren zur enantioselektiven enzymatischen Reduktion einer Ketoverbindung zur entsprechenden chiralen Hydroxyverbindung, wobei die Ketoverbindung in Gegenwart eines Co-Faktors mit einer Oxidoreduktase reduziert wird, dadurch gekennzeichnet, daß eine Oxidoreduktase eingesetzt wird, die eine Aminosäuresequenz aufweisen, bei welcher

(a) mindestens 70% der Aminosäuren identisch sind mit den Aminosäuren einer der Aminosäuresequenzen SEQ ID No 1, SEQ ID No 6 und SEQ ID No 8, oder
(b) mindestens 55% der Aminosäuren identisch sind mit den Aminosäuren der Aminosäuresequenz SEQ ID No 2, oder
(c) mindestens 65% der Aminosäuren identisch sind mit den Aminosäuren der Aminosäuresequenz SEQ ID No 3, oder
(d) mindestens 75% der Aminosäuren identisch sind mit den Aminosäuren der Aminosäuresequenz SEQ ID No 4, oder
(e) mindestens 65% der Aminosäuren identisch sind mit den Aminosäuren der Aminosäuresequenz SEQ ID No 5, oder
(f) mindestens 50 % der Aminosäuren identisch sind mit den Aminosäuren der Aminosäuresequenz SEQ ID No 7.
(g) mindestens **72** % der Aminosäuren identisch sind mit den Aminosäuren der Aminosäuresequenz SEQ ID No 129.

Eine weitere bevorzugte Ausführungsform des erfindungsgemäßen Verfahrens besteht darin, daß die Ketoverbindung die allgemeine Formel I

$$R_1\text{-C(O)-}R_2 \qquad (I)$$

aufweist, in der R1 für einen der Reste

1) -($C_1$-$C_{20}$)-Alkyl, worin Alkyl geradkettig oder verzweigtkettig ist,
2) -($C_2$-$C_{20}$)-Alkenyl, worin Alkenyl geradkettig oder verzweigtkettig ist und gegebenenfalls bis zu vier Doppelbindungen enthält,
3) -($C_2$-$C_{20}$)-Alkinyl, worin Alkinyl geradkettig oder verzweigtkettig ist und gegebenenfalls bis zu vier Dreifachbindungen enthält,
4) -($C_6$-$C_{14}$)-Aryl,
5) -($C_1$-$C_8$)-Alkyl-($C_6$-$C_{14}$)-Aryl,
6) -($C_5$-$C_{14}$)-Heterocyclus, der unsubstituiert oder ein-, zwei- oder dreifach durch -OH, Halogen, -$NO_2$ und/oder

$-NH_2$ substituiert ist, oder
7) $-(C_3-C_7)$-Cycloalkyl,

steht, wobei die oben unter 1) bis 7) genannten Reste unsubstituiert sind oder unabhängig voneinander ein-, zwei- oder dreifach durch -OH, Halogen, $-NO_2$ und/oder $-NH_2$ substituiert sind,
und $R_2$ für einen der Reste

8) $-(C_1-C_6)$-Alkyl, worin Alkyl geradkettig oder verzweigtkettig ist,
9) $-(C_2-C_6)$-Alkenyl, worin Alkenyl geradkettig oder verzweigtkettig ist und gegebenenfalls bis zu drei Doppelbindungen enthält,
10) $-(C_2-C_6)$-Alkinyl, worin Alkinyl geradkettig oder verzweigtkettig ist und gegebenenfalls zwei Dreifachbindungen enthält, oder
11) $-(C_1-C_{10})$-Alkyl-C(O)-O-$(C_1-C_6)$-Alkyl, worin Alkyl gerade oder verzweigtkettig ist und unsubstituiert ist oder ein-, zwei- oder dreifach durch -OH, Halogen, $-NO_2$ und/oder $-NH_2$ substituiert ist,

steht, wobei die oben unter 8) bis 11) genannten Reste unsubstituiert sind oder unabhängig voneinander ein-, zwei- oder dreifach durch -OH, Halogen, $-NO_2$ und/oder $-NH_2$ substituiert sind,
stehen.

Die Erfindung betrifft ferner ein Verfahren zur enantioselektiven enzymatischen Reduktion einer Ketoverbindung zur entsprechenden chiralen Hydroxyverbindung, wobei die Ketoverbindung in Gegenwart eines Co-Faktors mit einer Oxidoreduktase reduziert wird, das dadurch gekennzeichnet, daß eine Oxidoreduktase eingesetzt wird, für welche

(a) eine Nukleinsäuresequenz aus der Gruppe SEQ ID No 9, SEQ ID No 10, SEQ ID No 11, SEQ ID No 12, SEQ ID No 13 und SEQ ID No 14,SEQ ID No 15,SEQ ID No 16 und SEQ ID No 130 codiert, oder für welche
(b) eine Nukleinsäuresequenz codiert, deren komplementärer Strang mit einer der in (a) genannten Nukleinsäureseqenzen unter hochstringenten Bedingungen hybridisiert.

Unter dem Begriff Aryl werden aromatische Kohlenstoffreste verstanden mit 6 bis 14 Kohlenstoffatomen im Ring. $-(C_6-C_{14})$-Arylreste sind beispielsweise Phenyl, Naphthyl, zum Beispiel 1-Naphthyl, 2-Naphthyl, Biphenylyl, zum Beispiel 2-Biphenylyl, 3-Biphenylyl und 4-Biphenylyl, Anthryl oder Fluorenyl. Biphenylylreste, Naphthylreste und insbesondere Phenylreste sind bevorzugte Arylreste. Unter dem Begriff "Halogen" wird ein Element aus der Reihe Fluor, Chlor, Brom oder Jod verstanden. Unter dem Begriff "$-(C_1-C_{20})$-Alkyl wird ein Kohlenwasserstoffrest verstanden, dessen Kohlenstoffkette geradkettig oder verzweigt ist und 1 bis 20 Kohlenstoffatome enthält beispielsweise Methyl, Ethyl, Propyl, Isopropyl, Butyl, tertiär-Butyl, Pentyl, Hexyl, Heptyl, Octyl, Nonenyl oder Decanyl. Unter dem Begriff "-$C_0$-Alkyl" wird eine kovalente Bindung verstanden.

Unter dem Begriff "$-(C_3-C_7)$-Cycloalkyl" werden cyclische Kohlenwasserstoffreste verstanden wie Cyclopropyl, Cylobutyl, Cyclopentyl, Cyclohexyl oder Cycloheptyl.

Der Begriff "$-(C_5-C_{14)}$-Heterocyclus" steht für einen monocyclischen oder bicyclischen 5-gliedrigen bis 14-gliedrigen heterocyclischen Ring, der teilweise gesättigt oder vollständig gesättigt ist. Beispiele für Heteroatome sind N, O und S. Beispiele für die Begriffe $-(C_5-C_{14})$-Heterocyclus sind Reste, die sich von Pyrrol, Furan, Thiophen, Imidazol, Pyrazol, Oxazol, Isoxazol, Thiazol, Isothiazol, Tetrazol, 1,2,3,5-Oxathiadiazol-2-Oxide, Triazolone, Oxadiazolone, Isoxazolone, Oxadiazolidindione, Triazole, welche durch F, -CN, $-CF_3$ oder - C(O)-O-$(C_1-C_4)$-Alkyl substituiert sind, 3-Hydroxypyrro-2,4-dione, 5-Oxo-1,2,4-Thiadiazole, Pyridin, Pyrazin, Pyrimidin, Indol, Isoindol, Indazol, Phthalazin, Chinolin, Isochinolin, Chinoxalin, Chinazolin, Cinnolin, -Carbolin und benz-anellierte, cyclopenta-, cyclohexa- oder cyclohepta-anellierte Derivate dieser Heterocyclen ableiten. Insbesondere bevorzugt sind die Reste 2- oder 3-Pyrrolyl, Phenylpyrrolyl wie 4- oder 5-Phenyl-2-pyrrolyl, 2-Furyl, 2-Thienyl, 4-Imidazolyl, Methyl-imidazolyl, zum Beispiel 1-Methyl-2-, -4- oder -5-imidazolyl, 1,3-Thiazol-2-yl, 2-Pyridyl, 3-Pyridyl, 4-Pyridyl, 2-, 3- oder 4-Pyridyl-N-oxid, 2-Pyrazinyl, 2-, 4- oder 5-Pyrimidinyl, 2-, 3- oder 5-Indolyl, substituiertes 2-Indolyl, zum Beispiel 1-Methyl-, 5-Methyl-, 5-Methoxy-, 5-Benzyloxy-, 5-Chlor- oder 4,5-Dimethyl-2-indolyl, 1-Benzyl-2- oder -3-indolyl, 4,5,6,7-Tetrahydro-2-indolyl, Cyclohepta[b]-5-pyrrolyl, 2-, 3- oder 4-Chinolyl, 1-, 3- oder 4-Isochinolyl, 1-Oxo-1,2-dihydro-3-isochinolyl, 2-Chinoxalinyl, 2-Benzofuranyl, 2-Benzo-thienyl, 2-Benzoxazolyl oder Benzothiazolyl oder Dihydropyridinyl, Pyrrolidinyl, zum Beispiel 2- oder 3-(N-Methylpyrrolidinyl), Piperazinyl, Morpholinyl, Thiomorpholinyl, Tetrahydrothienyl oder Benzodioxolanyl.

Bevorzugte Verbindungen der Formel I sind beispielsweise Ethyl-4-chloracetoacetat, Methylacetoacetat, Ethyl-8-chloro-6-oxooctansäure, Ethyl-3-oxovaleriat, 4-Hydroxy-2-butanon, Ethyl-2-oxovaleriat, Ethyl-2-oxo-4-phenylbuttersäure, Ethylpyruvat, Ethylphenylglyoxylat, 1-Phenyl-2-propanon, 2-chloro-1-(3-chlorphenyl)ethan-1-on, Acetophenon, 2-Octanon, 3-Octanon, 2-Butanon, 1-[3,5-bis(trifluoromethyl)phenyl]ethan-1-one, 2,5-Hexandion, 1,4-Dichlor-2-butanon, Acetoxyaceton, Phenacylchlorid, Ethyl-4-bromoacetoacetat, 1,1-Dichloraceton, 1,1,3-Trichloraceton oder 1-Chloraceton.

Die Oxidoreduktasen können in dem erfindungsgemäßen Verfahren entweder vollständig gereinigt oder teilweise gereinigt eingesetzt werden oder mit Zellen, enthaltend die erfindungsgemäßen Oxidoreduktasen, durchgeführt werden. Die eingesetzten Zellen können dabei nativ, permeabilisiert oder lysiert vorliegen. Bevorzugt werden die klonierten Oxidoreduktasen gemäß SEQ ID No 1, SEQ ID No 2, SEQ ID No 3, SEQ ID No 4, SEQ ID No 5, SEQ ID No 6, SEQ ID No 7, SEQ ID No 8, SEQ ID No 129 bzw. deren Homologe eingesetzt.

Je kg umzusetzender Verbindung der Formel I werden 5 000 bis 10 Mio U Oxidoreduktase eingesetzt. (nach oben offen) Der Enzymeinheit 1 U entspricht dabei der Enzymmenge die benötigt wird um 1 $\mu$ mol der Verbindung der Formel I je Minute (min) umzusetzen

Die enzymatische Reduktion selbst läuft unter milden Bedingungen ab, so dass die erzeugten Alkohole nicht weiterreagieren. Die erfindungsgemäßen Verfahren weisen eine hohe Standzeit und eine Enantiomerenreinheit von in der Regel mehr als 95% der hergestellten chiralen Alkohole und eine hohe Ausbeute bezogen auf die eingesetzte Menge an Ketoverbindungen auf.

Die Carbonylverbindung wird im erfindungsgemäßen Verfahren in einer Menge von 3% bis 50% bezogen auf das Gesamtvolumen eingesetzt, bevorzugt von 5% bis 40%, insbesondere von 10%- 30%.

Eine bevorzugte Ausführungsform der Erfindung ist weiterhin dadurch gekennzeichnet, dass das bei der Reduktion gebildete NAD oder NADP kontinuierlich mit einem Cosubstrat zu NADH bzw. NADPH reduziert wird.

Als Cosubstrat werden dabei bevorzugt primäre und sekundäre Alkohole, wie Ethanol, 2-Propanol, 2-Butanol, 2-Pentanol, 3-Pentanol, 4-Methyl-2-pentanol, 2-Heptanol, 2-Octanol oder Cyclohexanol eingesetzt.

Diese Cosubstrate werden mit Hilfe einer Oxidoreduktase und NAD bzw. NADP zu den entsprechenden Aldehyden oder Ketonen und NADH bzw. NADPH umgesetzt. Dadurch kommt es zur Regenerierung des NADH bzw. NADPH. Der Anteil des Cosubstrates für die Regenerierung beträgt dabei von 5 bis 95 Vol%, bezogen auf das Gesamtvolumen.

Zur Regenerierung des Cofactors kann zusätzlich eine Alkoholdehydrogenase zugesetzt werden. Geeignete NADH-abhängige Alkoholdehydrogenasen sind beispielsweise erhältlich aus Bäckerhefe, aus Candida boidinii, Candida parapsilosis oder Pichia capsulata. Geeignete NADPH-abhängige Alkoholdehydrogenasen kommen ferner vor in Lactobacillus brevis (DE 196 10 984 A1), Lactobacillus minor (DE 101 19 274), Pseudomonas (US 5,385,833) oder in Thermoanaerobium brockii. Geeignete Cosubstrate für diese Alkoholdehydrogenasen sind die bereits genannten sekundären Alkohole wie Ethanol, 2-Propanol (Isopropanol), 2-Butanol, 2-Pentanol , 4-Methyl-2-pentanol, 2-Octanol oder Cyclohexanol.

Ferner kann die Cofactorregenerierung beispielsweise auch mit mittels NAD- oder NADPabhängiger Formiat-Dehydrogenase (Tishkov et al., J. Biotechnol. Bioeng. [1999] 64, 187-193, Pilot-scale production and isolation of recombinant NAD and NADP specific Formate dehydrogenase) durchgeführt werden. Geeignete Cosubstrate der Formiat-Dehydrogenase sind beispielsweise Salze der Ameisensäure wie Ammoniumformiat, Natriumformiat oder Calciumformiat. Bevorzugt werden die erfindungsgemäßen Verfahren jedoch ohne eine solche zusätzliche Dehydrogenase durchgeführt, d.h. es findet eine substratgekoppelte Coenzymregenerierung statt.

Der wässrige Anteil des Reaktionsgemisches, in dem die enzymatische Reduktion abläuft enthält bevorzugt einen Puffer, beispielsweise Kaliumphosphat-, Tris/HCl- oder Triethanolamin-Puffer, mit einem pH-Wert von 5 bis 10, vorzugsweise ein pH-Wert von 6 bis 9. Der Puffer kann zusätzlich noch Ionen zur Stabilisierung oder Aktivierung der Enzyme enthalten, beispielsweise Zinkionen oder Magnesiumionen.

Die Temperatur beträgt während der Durchführung der erfindungsgemäßen Verfahren zweckmäßig von etwa 10˚C bis 70˚C, bevorzugt von 20˚C bis 40˚C.

In einer weiteren bevorzugten Ausführungsform der erfindungsgemäßen Verfahren wird die enzymatische Umsetzung in Anwesenheit eines mit Wasser nicht oder nur beschränkt mischbaren organischen Lösungsmittels durchgeführt. Dieses Lösungsmittel ist beispielsweise ein symmetrischer oder unsymmetrischer Di($C_1$-$C_6$)alkylether, ein geradkettiges oder verzweigtes Alkan oder Cycloalkan oder ein wasserunlöslicher sekundärer Alkohol, der zugleich das Cosubstrat darstellt. Die bevorzugten organischen Lösungsmittel sind beispielsweise Diethylether, tertiär-Butylmethylether, Diisopropylether, Dibutylether, Butylacetat, Heptan, Hexan, 2-Oktanol, 2-Heptanol, 4-Methyl-2-pentanol oder Cyclohexan. Das Lösungsmittel kann dabei auch gleichzeitig als Cosubstrat zu Cofaktorregenerierung dienen.

Der Reaktionsansatz besteht beim Einsatz wasserunlöslicher Lösungsmittel bzw. Cosubstrate aus einer wässrigen und einer organischen Phase. Das Substrat verteilt sich entsprechend seiner Löslichkeit zwischen organischer und wässriger Phase. Die organische Phase hat allgemein einen Anteil von 5 bis 95%, bevorzugt von 10 bis 90% bezogen auf das gesamte Reaktionsvolumen. Die zwei flüssigen Phasen werden bevorzugt mechanisch gemischt, so dass eine große Oberfläche zwischen ihnen erzeugt wird. Auch in dieser Ausführungsform kann das bei der enzymatischen Reduktion gebildete NAD bzw. NADP mit einem Cosubstrat, wie beschrieben, wieder zu NADH bzw. NADPH reduziert werden.

Die Konzentration des Cofaktors NADH bzw. NADPH in der wässrigen Phase beträgt allgemein 0,001 mM bis 1mM, insbesondere 0,01 mM bis 0,1 mM.

In den erfindungsgemäßen Verfahren kann noch ein Stabilisator der Oxidoreduktase/Dehydrogenase eingesetzt werden. Geeignete Stabilisatoren sind beispielsweise Glycerin, Sorbitol, 1,4 -DL-Dithiothreit (DTT) oder Dimethylsulfoxid (DMSO).

Das erfindungsgemäße Verfahren wird beispielsweise in einem geschlossen Reaktionsgefäß aus Glas oder Metall durchgeführt. Dazu werden die Komponenten einzeln in das Reaktionsgefäß überführt und unter einer Atmosphäre von beispielsweise Stickstoff oder Luft gerührt. Die Reaktionszeit beträgt von 1 Stunde bis 48 Stunden, insbesondere von 2 Stunden bis 24 Stunden.

Anschließend wird das Reaktionsgemisch aufgearbeitet. Dazu wird die wässrige Phase abgetrennt, die organische Phase wird filtriert. Die wässrige Phase kann gegebenenfalls noch einmal extrahiert werden und wie die organische Phase weiter aufgearbeitet werden. Danach wird gegebenenfalls das Lösungsmittel aus der filtrierten organischen Phase verdampft.

Die Erfindung betrifft ferner ein Verfahren zur Gewinnung von chiralen Hydroxyverbindung der Formel II,

R1-C(OH)-R2 (II)

wobei $R^1$ und $R^2$ wie oben definiert sind, das dadurch gekennzeichnet ist, dass man

a) ein Gemisch, enthaltend die racemische Verbindung der Formel II, in Anwesenheit einer der erfindungsgemäßen Oxidoreduktasen gemäß SEQ ID No 1, SEQ ID No 2, SEQ ID No 3, SEQ ID No 4, SEQ ID No 5, SEQ ID No 6, SEQ ID No 7, SEQ ID No 8, SEQ ID No 129 oder deren Homologen , NAD(P) und Wasser inkubiert, wobei ein Enantiomer der Hydroxyverbindung der Formel II zur entsprechenden Ketoverbindung und NAD(P)H umgesetzt wird, und

b) das verbliebene Enantiomer der Hydroxyverbindung der Formel II isoliert.

Bei Einsatz der Carbonylreduktasen gemäß SEQ ID No 1, SEQ ID No 3, SEQ ID No 5, SEQ ID No 6 , SEQ ID No 7, SEQ ID No 129 erhält man dabei bevorzugt die entsprechenden chiralen R-Hydroxyverbindungen. Bei Einsatz der Carbonylreduktasen gemäß SEQ ID No 2, SEQ ID No 4 und SEQ ID No 8, erhält man dabei bevorzugt die entsprechenden chiralen S-Hydroxyverbindungen.

Die Reaktionsbedingungen sind im wesentlichen dieselben wie im obengenannten Verfahren zur enantiospezifischen Reduktion der Ketoverbindung der Formel I. Es wird jedoch statt einer enantioselektiven Reduktion der Ketoverbindung der Formel I aus dem racemischen Gemisch der Verbindung der Formel II nur ein Enantiomer der Hydroxyverbindung der Formel II enantioselektiv zur entsprechenden Ketoverbindung oxidiert. Dadurch verbleibt das entgegengesetzte Enantiomer der Hydroxyverbindung der Formel II und kann isoliert werden. Ferner wird im Verfahren anstelle der als Cosubstrate eingesetzten Alkohole wie Ethanol, 2-Propanol (Isopropanol), 2-Butanol, 2-Pentanol oder 2-Octanol, deren entsprechenden Ketone wie Aceton zur Regenerierung des NAD eingesetzt. Beispielsweise wird das Aceton und NAD(P)H mit der erfindungsgemäßen Oxidoreduktase oder einer zusätzlichen Dehydrogenase zu NAD und Isopropanol umgesetzt.

Bevorzugte Ausführungsformen der Erfindung werden mit den folgenden Beispiele noch näher erläutert.

*Beispiel 1:*

*Anzucht von Organismen und Sreening nach Carbonylreduktaseaktivität*

Zum Screening wurden die Hefestämme Rhodotorula mucilaginosa DSMZ 70825, Pichia farinosa DSMZ 3316, Candida nemodendra DSMZ 70647, Pichia stipidis DSMZ 3651 und Pichia trehalophila DSMZ 70391, Lodderomyces elongisporus DSMZ 70320 in folgendem Medium kultiviert: Hefeextrakt (3), Malzextrakt (3), Peptone (5) und Glucose (10) (Zahlen in Klammern sind jeweils g/l). Das Medium wurde bei 121˚C sterilisiert und die Hefen wurden ohne weitere pH-Regulierung bei 25˚C und auf einem Schüttler bei 160 Umdrehungen pro Minute (rpm) kultiviert.

Der Stamm Leuconostoc carnosum DSMZ 5576 wurde in folgendem Medium kultiviert: Glucose (20), Hefeextrakt (5), Fleischextrakt (10), Di-Ammoniumhydrogencitrat (2), Natriumacetat (5), Magnesiumsulfat (0,2), Mangansulfat (0,05), Di-Kaliumhydrogenphosphat (2). Das Medium wurde bei 121˚C sterilisiert und der Organismus wurde bei 30˚C ohne weitere pH-Regulierung oder Sauerstoffzufuhr kultiviert.

Der Stamm Microbacterium spec. DSMZ 20028 wurde auf einem Medium aus Hefeextrakt (3) und Trypticase Sojamehl (30) bei 30˚C und 160 Umdrehungen pro Minute (rpm) kultiviert.

Der Stamm Gordonia rubripertincta DSMZ 43570 wurde auf einem Medium aus Hefeextrakt (4), Glucose (4), Malzextrakt (10) und $CaCO_3$ (2) bei 37˚C und 160 Umdrehungen pro Minute (rpm) kultiviert.

Anschließend wurden 125 mg Zellen mit 800 µl Aufschlusspuffer (100 mM Triethanolamin (TEA), pH = 7,0) resuspendiert, mit 1 g Glasperlen versetzt und 10 Minuten (min) bei 4˚C in der Kugelmühle aufgeschlossen (Retsch). Der nach 2 min Zentrifugation bei 12000 rpm erhaltene Überstand (Lysat) wurde im folgenden Aktivitätsscreening und zur Bestimmung des Enatiomerenüberschusses (ee-Wert) eingesetzt. Als Substrate wurden verschiedene Ketone, wie 2-Butanone, 2-Oktanon, Ethyl-4-chloracetoacetat, Acetophenon oder Ethyl-2-oxo-4-phenylbuttersäure verwendet.

Ansatz fürs Aktivitätsscreening:

860 µl 0,1 M $KH_2PO_4/K_2PO_4$pH = 7,0 1mM $MgCl_2$
20 µl NADPH oder NADH (10 mM)
20 µl Lysat
100 µl Substrat (100 mM)

Die Reaktion wurde 1 min bei 340 nm verfolgt.

Ansatz für ee-Wert Bestimmung:

20 µl Lysat
100 µl NADH oder NADPH (50 mM)
60 µl Substrat (100 mM)

Die Ansätze zur ee-Bestimmung wurden nach 24 Stunden (h) beispielsweise mit Chloroform extrahiert und mittels Gaschromatographie (GC) wurde der Enantiomerenüberschuss bestimmt. Der Enantiomerenüberschuss wird wie folgt berechnet:

$$ee(\%) = ((\text{R-Alkohol -S-Alkohol})/(\text{R-Alkohol+ S-Alkohol})) \times 100.$$

Tabelle 1

| DSMZ Nr. | Mikrorganismus | Aktivität in U/g Zellen Wirtsorganismus | | | |
|---|---|---|---|---|---|
| | | 2-Butanon | | 2-Oktanon | |
| | | NADH | NADPH | NADH | NADPH |
| 70825 | Rhodotorula mucilaginosa | <1 | ----- | <1 | ----- |
| 3316 | Pichia farinosa | 12 | ----- | <1 | ----- |
| 70647 | Candida nemodendra | 45 | ----- | 12 | ----- |
| 3651 | Pichia stipidis | 10 | ----- | 6,4 | ----- |
| 70391 | Pichia trehalophila | 85 | ----- | 45 | ----- |
| 5576 | Leuconostoc carnosum | ----- | 77 | ----- | 77 |
| 20028 | Microbacterium spec. | 9 | ----- | 26 | ----- |
| 43570 | Gordonia rubripertincta | 7,7 | ----- | 13 | ----- |
| 70320 | Lodderomyces elogisporus | 40 | ----- | 34 | ----- |

DSMZ steht für Deutschen Sammlung für Mikroorganismen und Zellkulturen, Mascheroder Weg 1b, 38124 Braunschweig. Definition der Enzymeinheiten: 1 U entspricht der Enzymmenge die benötigt wird um 1 µmol Substrat pro min umzusetzen.

*Beispiel* 2:

*Isolierung und Reinigung von NAD(P)H abhängigen mikrobiellen Oxidoreduktasen*

Zur Isolierung der NAD(P)H abhängigen mikrobiellen Oxidoreduktasen wurden die Mikrorganismen wie unter Beispiel 1 beschrieben kultiviert. Nach Erreichen der stationären Phase wurden die Zellen geerntet und durch Zentrifugation vom Medium getrennt. Die Enzymfreisetzung erfolgte durch Naßvermahlung mittels Glasperlen, kann aber auch durch andere Aufschlussmethoden erreicht werden. Dazu wurden beispielsweise 100 g Zellfeuchtmasse mit 400 ml Aufschlusspuffer (100 mM Trieethanolamin, 1 mM $MgCl_2$, pH =7,0) suspendiert und mittels einer French press homogenisiert.

Der nach Zentrifugation (7000 rpm) erhaltene Rohextrakt wurde dann mittels FPLC (fast protein liquid chromatography) weiter aufgereinigt.

Alle erfindungsgemäßen Oxidoreduktasen konnten durch unterschiedliche Kombinationen von Ionenaustauschchromatographie, beispielsweise an Q-Sepharose Fast Flow (Pharmacia) oder Uno Q (Biorad, München, Deutschland), hydrophober Interaktionschromatographie, beispielsweise an Octylsepharose Fast Flow oder Butylsepharose Fast Flow (Pharmacia), Keramik-Hydroxyapatit-Chromatographie und Gelpermataion aufgereinigt werden.

*Beispiel 2a:*

*Reinigung einer NADH abhängigen Oxidoreduktase aus Pichia farinosa DSMZ 3316*

Zur Proteinisolierung wurde das nach Zentrifugation erhaltene Lysat aus *Pichia farinosa DSMZ 3316* direkt auf eine mit 100 mM Triethanolaminpuffer pH = 7,0 1 M $(NH_4)_2SO_4$ äquilibrierte Butylsepharose FF-Säule aufgetragen und mit fallendem linearen Salzgradienten eluierte. Die oxidoreduktase-enthaltenden Fraktionen wurden vereinigt und mittels Ultrafiltration (Ausschlussgrenze 10 kDa) auf ein geeignetes Volumen eingeengt.

Anschließend wurde die eingeengten Fraktionen der Oxidoreduktase mittels Uno Q weiter aufgereinigt. Dazu wurde die Oxidoreduktase direkt auf eine mit 50 mM Kaliumphosphatpuffer pH = 7,0 äquilibrierte UnoQ-Säule (Biorad) aufgetragen und mit steigendem linearen Salzgradienten eluiert, wobei die Oxidoreduktase ohne Bindung bei 0 M NaCl eluierte während ein Großteil der Verunreinigungen gebunden wurde und bei höheren Salzkonzentrationen eluierte.

Der dritte Reinigungsschritt wurde an einer Keramik Hydroxyapatit Säule (Pharmacia) durchgeführt, wobei die Oxidoreduktase auf eine mit 10 mM Kaliumphosphatpuffer, 1mM $MgCl_2$ pH = 6,8 äquilibrierte Säule aufgetragen und mit steigender Pufferkonzentration (400 mM Kaliumphosphatpuffer 1mM $MgCl_2$ pH = 6,8 eluiert wurde. Die Oxidoreduktase wurde bei 80-100 mM Kaliumphosphatpuffer eluiert.

Danach wurde das Molekulargewicht der erhaltenen aufgereinigten Oxidoreduktase mit Hilfe von Gelpermeation (Superdex 200 HR; Pharmacia, 100 mM Trieethanolamin, pH = 7, 0,15 M NaCl) bestimmt. Als Molekulargewichtsstandards wurden Catalase (232 kDa), Aldolase (158 kDa), Albumin (69,8kDa) und Ovalbumin (49,4 kDa) verwendet.

Die folgende Tabelle 2 fasst die erhaltenen Ergebnisse zusammen

Tabelle 2:

| Reinigungsschritt | Volumen [ml] | Aktivität [U/ml] 2-Butanon | Gesamtaktivität [U] 2-Butanon | Spezifische Aktivität [U/mg] 2-Butanon | Ausbeute |
|---|---|---|---|---|---|
| Rohextrakt | 70 | 1,3 | 80 | 0,1 | 100% |
| Butylsepharose | 10 | 4,4 | 44 | 1,7 | 55% |
| Uno Q | 1,4 | 17 | 24 | 5 | 30% |
| Hydroxyapatit | 0,7 | 13,5 | 9,5 | 16 | 12% |

Die Enzymaktivität der Oxidoreduktase wurde im Testsystem gemäß Beispiel 1, (Ansatz Aktivitätsscreening) bestimmt und die Bestimmung der Proteinmenge erfolgte gemäß Lowry et al. Journal of Biological Chemistry, 193 (1951): 265-275 oder Peterson et al., Analytical Biochemistry, 100 (1979): 201-220). Der Quotient von Enzymaktivität zu Proteinmenge ergibt die spezifische Aktivität, wobei der Umsatz von 1 μmol pro min 1 Unit (U) entspricht.

*Beispiel 2b:*

*Reinigung einer NADH abhängigen Oxidoreduktase aus Microbacterium spec. DSMZ 20028*

Zur Proteinisolierung wurde das nach Zentrifugation erhaltene Lysat aus *Microbacteriun spec. DSMZ 20028* auf eine mit 50 mM Kaliumphosphatpuffer pH = 7,0 äquilibrierte Q-Sepharose FF-Säule aufgetragen und mit steigendem linearen Salzgradienten eluierte. Die Oxidoreduktase wurde dabei bei 0,6 bis 0,8 M NaCl eluiert. Die Oxidoreduktase-enthaltenden Fraktionen wurden vereinigt und mittels Ultrafiltration (Ausschlussgrenze 10 kDa) auf ein geeignetes Volumen eingeengt.

Anschließend wurden die eingeengten Fraktionen der Oxidoreduktase mittels Uno Q weiter aufgereinigt. Dazu wurde die Oxidoreduktase direkt auf eine mit 50 mM Kaliumphosphatpuffer pH = 7,0 äquilibrierte UnoQ-Säule (Biorad) aufgetragen und mit steigendem linearen Salzgradienten eluiert, wobei die Oxidoreduktase bei 0,2-0,25 M NaCl eluierte.

Der dritte Reinigungsschritt wurde an einer Keramik Hydroxyapatit Säule (Pharmacia) durchgeführt wobei die Oxidoreduktase aus *Microbacterium spec. DSMZ 20028* auf eine mit 10 mM Kaliumphosphatpuffer, 1mM $MgCl_2$ pH = 6,8 äquilibrierte Säule aufgetragen und mit steigender Pufferkonzentration (400 mM Kaliumphosphatpuffer 1mM $MgCl_2$ pH = 6,8) eluiert wurde. Die Oxidoreduktase wurde bei 80-100 mM Kaliumphosphatpuffer eluiert. Danach wurde das Molekulargewicht der erhaltenen aufgereinigten Oxidoreduktase wurde wie unter 2a beschrieben bestimmt.

Die folgende Tabelle 3 fasst die erhaltenen Ergebnisse zusammen

Tabelle 3:

| Reinigungsschritt | Volumen [ml] | Aktivität [U/ml] 2-Oktanon | Gesamtaktivität [U] 2-Oktanon | Spezifische Aktivität [U/mg] 2-Oktanon | Ausbeute |
|---|---|---|---|---|---|
| Rohextrakt | 55 | 3,8 | 212 | 0,4 | 100% |
| Q-Sepharose FF | 34 | 4,1 | 139 | 0,56 | 65% |
| Uno Q | 0,8 | 9,3 | 7,5 | 3,8 | 3,5% |
| Hydroxyapatit | 0.5 | 4,2 | 2,1 | 117 | 1% |

*Beispiel 3:*

*Bestimmung der N-terminalen Sequenz einer erfindungsgemäßen Oxidoreduktase*

Die Enzympräparationen nach Beispiel 2 wurden nach der Gelpermeation im 10% igen Natriumdodecylsulfat (SDS) Gel aufgetrennt und auf eine Polyvinyliden Diflourid-Membran (PVDF-Membran) übertragen.

Die auffällige Bande wurde einer N-terminalen Sequenzierung mittels Edman-Abbau (Procise 492 (PE-Biosystems) unterworfen.

*Beispiel 4:*

*Allgemeine Klonierungsstrategie einer enantioselektiven Alkoholdehydrogenase isoliert aus Hefen*

Chromosomale DNA wird nach der in "Molecular cloning" von Manniatis & Sambrook beschriebenen Methode extrahiert. Die resultierende Nukleinsäure dient als Matrize für die Polymerasen Kettenreaktion (PCR) mit degenerierten Primern. Dabei werden 5'-Primer von der Aminosäurensequenz (SEQIDNo. 66; 72; 80) und die 3'-Primer von der Aminosäuresequenz (SEQIDNo. 67; 73, 81) unter Einbeziehung des für Organismus spezifischen Genkodes abgeleitet (SEQIDNo. 68; 69; 74; 75; 82; 83).

Die Amplifizierung wird in PCR-Puffer [67mM Tris-HCl (pH 8.3), 16 mM $(NH_4)_2SO_4$, 115 mM $MgCl_2$, 0.01% Tween 20], 0.2 mM Desoxy-Nukleotidtriphosphat Mix (dNTPs), 40 pMol je Primer und 2.5 U BioTherm Star Polymerase (Genecraft, Lüdingshausen, Deutschland)] durchgeführt. Nach einer Aktivierung der BioTherm Star Polymerase (8 min 95°C) und folgenden 45-50 Zyklen einer Touch-Down PCR wird die Reaktion auf 4°C abgekühlt und der gesamte PCR-Ansatz auf ein 1% Agarose Gel zur Analyse aufgetragen.

Das spezifische Fragment, das der Polymerase Kettenreaktion resultiert, wird in den TA-Kloningsvektor pCR2.1 (Invitrogen, Karlsruhe, Deutschland) ligiert und mit den Primern M13 rev (SEQIDNo 65) und M13 uni (SEQIDNo 128) mit Hilfe von ABI DNA Sequenzer sequenziert.

Die 5'- und 3'-terminale Bereiche der Gen kodierenden Sequenz werden mit Hilfe von RACE Methode (rapid amplification of cDNA ends) bestimmt. Basierend auf der Nukleinsäuresequenz des spezifischen Fragments werden Oligonukleotide für 3'-RACE und 5'-RACE konstruiert. Als Matrize für die Synthese vom ersten cDNA Strang mit Hilfe von 3'-RACE System (Invitrogen, Karlsruhe, Deutschland) dient aus den Zellen präparierte gesamte RNA. Es folgen eine Amplifikation und eine Reamplifikation des spezifischen cDNA mit Hilfe von 3'-RACE Oligonukleotiden (SEQIDNo. 76; 77; 84; 85). Anschließend wird der Ansatz zur Analyse auf ein 1% Agarose Gel aufgetragen. Das spezifische Fragment, das die fehlende 3'-flankierende Sequenzinformation trägt, wird isoliert in einen TA-Kloningsvektor pCR2.1 ligiert und sequenziert.

Die kodierenden und nicht kodierenden 5'-terminalen Sequenzen werden mit Hilfe von 5'-RACE System (Invitrogen) bestimmt. Dazu wird mit Hilfe von Oligo dT-Cellulose (NEB, Beverly, USA) mRNA aus der zuvor gewonnenen Gesamt-RNA angereichert und in die Synthese des ersten cDNA Stranges mit den genspezifischen Oligonukleotiden (SEQIDNo. 70; 71; 78; 79; 86; 87) angesetzt. Nachfolgende Amplifikation und Reamplifikation der spezifischen cDNA resultiert in einem Fragment, das zur Analyse in ein pCR2.1 TA-Kloningsvektor (Invitrogen) ligiert wird. Das das Fragment enthaltende Plasmid wird mit Hilfe eines ABI DNA Sequenzer analysiert. Somit werden die fehlende Sequenzinformationen über 5'-Ende des Gens gewonnen.

| Protein | Rhodotorulla mucilaginosa | Pichia farinosa | Pichia stipitis |
|---|---|---|---|
| Ansequenzierte Peptide | VATAVETFGR (SEQIDNo 66)<br>FGEAVEQAR (SEQIDNo 67) | LLTQTLALEQAK (SEQIDNo 72)<br>YNFTNKVAIITGGI (SEQIDNo 73) | ADQVLLK (SEQIDNo 80)<br>ISFNLGDLALR (SEQIDNo 81) |
| Primer für Touch-Down PCR | CCRAAYTCVACVGCVGTSGC (SEQIDNo 68)<br>GCCTGYTCGACVGCYTCRCC (SEQIDNo 69) | YTGYTCYAANGCYAADGTYTG (SEQIDNo 74)<br>CHAAYAARGTNGCHATHATYAC HGG (SEQIDNo 75) | GCYGAYCARGTNTTRTTRA AR (SEQIDNo 82)<br>CTYAARGCYAARTCDCCYA AR (SEQIDNo 83) |
| Primer für 3'-RACE | | CAACGTTCTGAAGAGATGACTTA TG (SEQIDNo 76)<br>GGTGGAGTGAAGTTATTGAC (SEQIDNo 77) | CTACCATGCCATGAGATTA G (SEQIDNo 84)<br>GCTGTAGACGTCGCTAAGA G (SEQIDNo 85) |
| Primer für 5'-RACE | CTCCGAGGTGTTGAGCGCAT TG (SEQIDNo 70)<br>GACGAGGTTCTTGATGTCGT CCTCC (SEQIDNo 71) | GCCATTCTTAGCCTGTTCGAGAG (SEQIDNo 78)<br>GTCATCTCTTCAGAACGTTGATC TT (SEQIDNo 79)<br>CCAAAGGAGCTTATAGCAGTCT (SEQIDNo 88) | GATTCTCAAGGCTAAGTCA C (SEQIDNo 86)<br>GATCTAACACCAGCTAATC T (SEQIDNo 87) |

Basierend auf der Sequenz, die für das Volllängen-Gen kodiert (SEQIDNo. 9; 10; 11) werden spezifische Primer für eine nachfolgende Klonierung dieses DNA Abschnittes in ein passendes Expressionssystem konstruiert. Dafür werden beispielsweise 5'-Primer mit einer Erkennungssequenz für Nde I, bzw. mit einer Erkennungssequenz für Sph I, bzw. für BamHI und 3' Primer mit einer Erkennungssquenz für Hind III modifiziert (SEQIDNo. 89; 90; 91; 92; 93; 94; 95; 96).

Chromosomale DNA dient in nachfolgender PCR als Matrize. Der für die jeweilige Oxidoreduktase kodierende DNA Abschnitt wird mit Hilfe von *Platinum pfx* Polymerase (Invitrogen) amplifiziert. Das resultierende PCR-Produkt wird nach der Reinigung über ein 1% Agarose Gel mit entsprechenden DNA Endonukleasen behandelt und in das mit den gleichen Endonukleasen behandelten Rückgrad des pET21a Vektors (Novagen, Madison, USA), bzw. Rückgrad des pQE70 Vektors (Qiagen, Hilden, Deutschland) ligiert.

Das entstandene Expressionskonstrukt wird nach der Sequenzierung in Expressionsstamm BL21 Star (Invitrogen), bzw. RB791 (E.coli genetic stock, Yale, USA) gebracht.

*4a. Klonierung einer enantioselektiven Oxidoreduktase aus Hefe Pichia Farinosa*

Zur Klonierung der Oxidoreduktase aus Pichia farinosa wurde beispielsweise chromosomale DNA aus den frischen Zellen Pichia Farinosa nach der in "Molecular cloning " von Manniatis & Sambrook beschriebenen Methode extrahiert. Die resultierende Nukleinsäure diente als Matrize für eine Touch-Down PCR mit Oligonukleotiden SEQIDNo. 74; 75. Nach einer 8 minutigen Aktivierung der Biotherm Star Polymerase in einem PCR Cycler (BioRad, Hercules, USA) wurden folgende 30 Temperatur-Zyklen für eine Identifizierung des spezifischen DNA Fragmentes programmiert:

| | |
|---|---|
| 94˚C | 45Sec |
| 60 ˚ C - 0.5 ˚ C / cycle | 45 Sec |
| 68 ˚ C | 2 min |

Anschließend wurde das Amplifizierungssignal durch weitere 20 Zyklen

| | |
|---|---|
| 94 ˚ C | 40 Sec |
| 52 ˚ C | 40 Sec |
| 72 ˚ C | 1 Min |

verstärkt. Nach der Auftrennung des gesamten Reaktionsansatzes in einem 1% Agarose Gel wurde ein spezifisches Fragment mit einer Größe von 550 bp detektiert. Dieses Fragment wurde aus dem Gel eluiert und in den pCR2.1 TA-Vektor (Invitrogen, Karlsruhe, Deutschland) ligiert. Das entstandene Plasmid pCR2.1-PF550 wurde einer Sequenzierung unterzogen.

Sequenzanalyse des 550 bp langen Genfragmentes zeigte einen offenen Leserrahmen von 174 Aminosäurenresten, in den auch beide Sequenz-Fragmente des N-Terminus und des internen Peptides wieder zu finden waren.

Basierend auf die Nukleotidsequenz des 521 bp langen Fragmentes wurden Oligonukleotide für eine 3'-RACE (SEQIDNo 76; 77) und 5'-RACE (SEQIDNo 78; 79; 88) konstruiert. Für die cDNA Synthese Reaktion wurde die gesamte RNA aus den Zellen Pichia farinosa wie folgt präpariert.

600 mg frische Zellen wurden in 2,5 ml eiskalten LETS Puffer resuspendiert. Zu dieser Zellsuspension wurden 5 ml (etwa 20 g) in Salpetersäure gewaschener Glasperlen, equilibriert mit 3 ml Phenol (pH 7.0), dazugegeben. Der gesamte Ansatz wurde dann insgesamt 10 min jeweils 30 sec gevortext und 30 sec auf Eis gekühlt. Anschließend wurden 5 ml eiskalten LETS Puffer dazugegeben und noch mal kräftig gevortext. Diese Zellsuspension wurde für 5 min bei 11000 g bei 4 ˚ C zentrifugiert. Die wässrige Phase wurde gewonnen und mit gleichem Volumen an Phenol: Chloroform: Isoamylalkohol (24:24:1) zweimal extrahiert. Anschließend es folgte die Extraktion mit Chloroform. Nach der letzten Extraktion wurde die gesamte RNA durch die Zugabe von 1 / 10 Vol an 5 M $LiCl_2$ bei -20 ˚ C 4 h präzipitiert.

Die Synthese von dem ersten cDNA Strang wurde mit Hilfe von 3'RACE System (Invitrogen, Karlsruhe, Deutschland) durchgeführt. Anschließend wurde die spezifische cDNA mit Oligonukleotiden SEQIDNo76 und AUAP (Invitrogen, Karlsruhe, Deutschland) in der Reaktion: 67mM Tris-HCl (pH 8.3), 16 mM $(NH_4)_2SO_4$, 115 mM $MgCl_2$, 0.01% Tween 20], 0.2 mM Desoxy-Nukleotidtriphosphat Mix (dNTPs), 10 pMolje Primer und 2.5 U BioTherm Star Polymerase (Genecraft, Lüdingshausen, Deutschland) und folgenden 30 Temperatur-Zyklen : 94 ˚ C 40 Sec, 55 ˚ C 40 Sec, 72 ˚ C 1 Min amplifiziert.

Das PCR-Signal wurde durch eine Nest-PCR mit Primer SEQIDNo 77 und Primer UAP (Invitrogen, Karlsruhe, Deutschland) mit 30 Temperatur-Zyklen: 94 ˚ C 40 Sec, 55 ˚ C 40 Sec, 72 ˚ C 50 Sec verstärkt. Es resultierte ein spezifisches DNA Fragment einer Größe von ungefähr 400 bp, das nach der Isolierung aus dem 1% Agarose Gel in den Vektor pCR2.1 (Invitrogen) ligiert wurde. Die Sequenzanalyse des 382 bp langen DNA Abschnittes lieferte Sequenzinformationen über die 3'-Verlängerung bis zu dem Stop-Kodon und der poly-A Schleife der für die Oxidoreduktase aus Pichia Farinosa kodierenden cDNA.

Für die 5'RACE Reaktion wurden 5 μg gesamt RNA präpariert aus den Zellen Pichia farinosa benutzt. Die Synthese von genspezifischer cDNA wurde mittels 5'RACE System (Invitrogen, Karlsruhe, Deutschland) und dem Oligonukleotid SEQIDNo 78 durchgeführt. Die resultierende genspezifische cDNA wurde einer homopolymerischen dCTP Addierungsreaktion unterzogen. Anschließend es folgte eine Amplifizierung der cDNA in einer PCR [67mM Tris-HCl (pH 8.3), 16 mM $(NH_4)_2SO_4$, 115 mM $MgCl_2$, 0.01% Tween 20], 0.2 mM Desoxy-Nukleotidtriphosphat Mix (dNTPs), 20 pmol Primer SEQIDNo 79 und Primer AAP (Invitrogen), 2.5 U BioTherm Star Polymerase (Genecraft, Lüdingshausen, Deutschland) und folgenden 35 Temperatur-Zyklen : 94 ˚ C 45 sec, 54 ˚ C 45 sec, 72 ˚ C 1 min 30 sec. Das PCR-Signal wurde durch eine Nest-PCR mit Primer SEQIDNo 88 und Primer UAP (Invitrogen, Karlsruhe, Deutschland) mit 30 Temperatur-Zyklen: 94 ˚ C 40 sec, 55 ˚ C 40 sec, 72 ˚ C 1 min verstärkt. Es resultierte ein spezifisches DNA Fragment einer Größe von ungefähr 350 bp, das nach der Elution aus dem 1% Agarose Gel in den Vektor pCR2.1 (Invitrogen) ligiert wurde. Die Sequenzanalyse des 352 bp langen DNA Segmentes lieferte Sequenzinformationen über 5'-Ende der für die Alkoholdehydrogenase / Reduktase kodierenden cDNA.

Somit besitzt das DNA Segment, das für das Protein kodiert, eine gesamt Länge von 765 bp (SEQIDNo 10), und einen offenen Leserrahmen von 254 Aminosäuren (SEQIDNo 2). Chromosomale DNA der Pichia farinosa Zellen wurde als Matrize für die Generierung der Volllängen-DNA in einer Polymerase Kettenreaktion [10 mM Tris-HCl, (pH 8.0); 50 mM KCl; 10 mM $MgSO_4$; 0.2 mM dNTP Mix; 20 pMol Primer SEQIDNo 91,bzw. 20 pMol Primer SEQIDNo 92, 20 pMol Primer SEQIDNo 93 und 2 U *Platinum pfx* Polymerase (Invitrogen)] und Temperatur-Zyklen:

| | |
|---|---|
| Zyklus 1 | 94 ˚C, 2 min |
| Zyklus 2 x 30 | 94 ˚C, 15 sec |
| | 56 ˚C, 20 sec |
| | 68 ˚C, 1min 15 sec |

verwendet.

Das resultierende PCR-Produkt wurde nach der Reinigung über 1% Agarose Gel mit Nde I und Hind III, bzw. mit Sph I und Hind III behandelt und in das mit den gleichen Endonukleasen behandelten Rückgrad des Vektors pET21a (Novagen, Madison, USA), bzw. pQE70 (Qiagen, Hilden, Deutschland) ligiert. Nach der Transformation von 2 μl des Ligationsansatzes in E.coli Top10F' Zellen wurden Plasmid-DNA's Ampicillin-resistenter Kolonien mittels einer Restriktionsanalyse mit Endonukleasen Nde I, bzw. Sph I und Hind III auf die Richtigkeit der erfolgten Ligation überprüft. Die DNA der für das Insert positiven Vektoren wurde in Expressionsstamm BL21 Star (Invitrogen), bzw. RB791 (E.coli genetic Stock, Yale, USA) transformiert.

*Beispiel 5:*

*Allgemeine Klonierungsstrategie einer enantioselektiven Oxidoreduktase isoliert aus Bakterien*

Genomische DNA wird nach der in "Molecular cloning" von Manniatis & Sambrook beschriebenen Methode extrahiert. Die resultierende Nukleinsäure dient als Matrize für die Polymerasen Kettenreaktion (PCR) mit degenerierten Primern. Dabei werden 5'-Primer von der Aminosäurensequenz (SEQIDNo 104; 112) und die 3'-Primer von der Aminosäuresequenz (SEQIDNo 105; 113) unter Einbeziehung des für Organismus spezifischen Genkodes abgeleitet (SEQIDNo 106; 107; 114; 115)

Die Amplifizierung wird in PCR-Buffer [67mM Tris-HCl (pH 8.3), 16 mM $(NH_4)_2SO_4$, 115 mM $MgCl_2$, 0.01% Tween 20], 0.2 mM Desoxy-Nukleotidtriphosphat Mix (dNTPs), 40 pMol je Primer und 2.5 U BioTherm Star Polymerase (Genecraft, Lüdingshausen, Deutschland)] durchgeführt. Nach einer Aktivierung der BioTherm Star Polymerase (8 min 95 ˚ C) und folgenden 45-50 Zyklen einer Touch-Down PCR wird die Reaktion auf 4 ˚ C abgekühlt und der gesamte PCR-Ansatz auf ein 1% Agarose Gel zur Analyse aufgetragen.

Das spezifische Fragment, das der Polymerase Kettenreaktion resultiert, wird in den TA-Kloningsvektor pCR2.1 (Invitrogen, Karlsruhe, Deutschland) ligiert und mit den Primern M13 rev (SEQIDNo 65) und M13 uni (SEQIDNo 128) mit Hilfe eines ABI DNA Sequenzer sequenziert.

5'-und 3'-flankierende Bereiche der Gen kodierenden Sequenz werden mit Hilfe von inversen Polymerase Kettenreaktionsmethode (iPCR) bestimmt. Basierend auf der Nukleinsäuresequenz des spezifischen internen Fragments werden Oligonukleotide SEQIDNo 100; 101; 102; 103; 108; 109; 110; 111; 116; 117; 118; 119 konstruiert. Genomische DNA wird mit Hilfe einer Restriktionsendonuklease verdaut und in eine Religation eingesetzt, so dass kleinere DNA Abschnitte zirkularisieren. Dieses Religationsgemisch wird dann als Matrize für eine iPCR und Primer SEQIDNo 100; 102; 108; 110; 116; 118 verwendet. Das PCR-Signal wird durch anschließende Nest-PCR mit Primer SEQIDNo 101; 103; 109; 111; 117; 119 verstärkt. Das resultierende spezifische Fragment wird nach der Elution aus dem 1% Agarose Gel in das Vektor pCR2.1 (Invitrogen) ligiert.

Somit liefert die Sequenzierungsanalyse des Fragment enthaltenden Vektors pCR2.1 die fehlende Sequenzinformationen über 3'- und 5'-kodierende Bereiche des Alkoholdehydrogenase / Reduktase Gens.

| Protein | Leuconostoc carnosum | Microbacterium sp. | Gordonia rubropertincta |
|---|---|---|---|
| Ansequenzierte Peptide | NIEETTYEDWK (SEQIDNo 97) | MKALQYTKIGSHPE (SEQIDNo 104) | MKAIQIIQPG (SEQIDNo 112)<br>VGPPTQPYEVSVR (SEQIDNo 113) |
| | | AYEALAAGTVV (SEQIDNo 105) | |
| Primer für Touch-Down PCR | GACAGAWMGWTTNAARGGWAARGTHGC (SEQIDNo 98)<br>GCBGTRTAWCCNCCRTCDACDACRAAYTC (SEQIDNo 99) | CTSCARTACACVAAGATCGG (SEQIDNo 106)<br>GCBGCSAGBGCYTCRTABGC (SEQIDNo 107) | ATGAARGCNATYCARATYATYCARCC (SEQIDNo 114)<br>CYTCRTANGGYTGNGTRAARAA (SEQIDNo 115) |
| Primer für iPCR | CTAAGCCAATACCAAGTGTACCA (SEQIDNo 100)<br>GAACAAATCGTGCTACTGATTCATCAC (SEQIDNo 101)<br>GAAGAAGCCCAATCACAAAGAACTC (SEQIDNo 102)<br>GGCAGTCTATTTAGCTAGTGAAG (SEQIDNo 103) | TCCTCGCTGAGGCTCATCAC (SEQIDNo 108)<br>GCTTCTCGATCTCGACGACTTC (SEQIDNo 109)<br>GCGCAGCGAACTGATCGAG (SEQIDNo 110)<br>GATCCAGCGCTACTCACTCGAC (SEQIDNo 111) | GAGGACGAAGTCGTCCGAATG (SEQIDNo 116)<br>GCCGTCACCTTCAGCAACACC (SEQIDNo 117)<br>CTCGACGTGAGCGACGACAAG (SEQIDNo 118)<br>GCAAGATCACCGGCAACGATG (SEQIDNo 119) |

Basierend auf der Sequenz, die für das Volllängen-Gen kodiert (SEQIDNo. 12; 13; 14) werden spezifische Primer für eine nachfolgende Klonierung dieses DNA Abschnittes in ein passendes Expressionssystem konstruiert. Dabei werden 5'-Primer mit einer Erkennungssequenz für Nde I, bzw. mit einer Erkennungssequenz für Sph I, bzw. für BamHI und 3' Primer mit einer Erkennungssqunez für Hind III modifiziert (SEQIDNo. 120; 121; 122; 123; 124; 125; 126; 127).

Die Amplifizierung der für das Protein kodierenden Volllängen-DNA aus genomischer DNA mit nachfolgender Restriktion und Ligation in den Expressionsvektor erfolgt wie im Beispiel 3 beschrieben. Der Expressionsstamm BL21 Star (Invitrogen), bzw. RB791 (E.coli genetic stock, Yale, USA) wird mit dem entstandenen Expressionskonstrukt transformiert.

*5a Klonierung einer enantioselektiven Alkoholdehydrogenase / Reduktase aus Microorganismus Microbacterium sp.*

Zur Klonierung der Oxidoreduktase aus Microbacterium sp. wurde beispielsweise genomische DNA aus den frischen Zellen Microbacterium sp. nach der in "Molecular cloning " von Manniatis & Sambrook beschriebenen Methode extrahiert. Die resultierende Nukleinsäure diente als Matrize für eine PCR mit je 30 pMol an Oligonukleotiden SEQIDNo. 106; 107. Nach einer 10 minutigen Aktivierung des Biotherm Star Polymerase in einem PCR Cycler (BioRad, Hercules, USA) wurden folgende 30 Temperatur-Zyklen für eine Identifizierung des spezifischen DNA Fragmentes programmiert:

| | |
|---|---|
| 94 ˚ C | 50 sec |
| 60 ˚ C | 1 min |
| 72 ˚ C | 1 min |

Nach der Auftrennung des gesamten Reaktionsansatzes in einem 1% Agarose Gel wurde ein spezifisches Fragment mit einer Größe von ungefähr 1000 bp detektiert. Dieses Fragment wurde aus dem Gel eluiert und in das pCR2.1 TA-Vektor (Invitrogen, Karlsruhe, Deutschland) ligiert. Das entstandene Plasmid pCR2.1-Ms 1000 wurde einer Sequenzierung unterzogen.

Sequenzanalyse des 1002 bp langen Genfragmentes zeigte einen offenen Leserrahmen von 334 Aminosäurenresten, in den auch beide Sequenz-Fragmente des N-Terminus und des internen Peptides wieder zu finden waren.

Basierend auf der Nukleotidsequenz des 1002 bp langen Fragmentes wurden Oligonukleotide (SEQIDNo 108; 109; 110; 111) für eine inverse PCR (iPCR) konstruiert.

Genomische DNA (2.5 μg) aus den Zellen Microbacterium sp. wurde in einem 50 μl Ansatz mit 20 U Restriktionsendonuklease Sac I 25 min behandelt. Nach der Phenol:Chloroform:Isoamylalkohol (25:24:1) Extraktion des gesamten Ansatzes und Fällung mit 1/10 Vol an 3M Na-Acetat (pH5.2) und 2.5 Vol an Ethanol wurde die somit verdaute DNA in 25 μl $H_2O$ aufgenommen. Davon 5 μl (200 ng) wurden in einer Religation Reaktion im gesamten Volumen von 40 μl und 2 U T4 Ligase (Fermentas) eingesetzt. Die religierte genomische DNA (2μl = 20 ng) wurde dann in eine iPCR [67mM Tris-HCl (pH 8.3), 16 mM $(NH_4)_2SO_4$, 115 mM $MgCl_2$, 0.01% Tween 20], 0.2 mM Desoxy-Nukleotidtriphosphat Mix (dNTPs), 30 pMol je Primer (SEQIDNo 108; 110) mit 2.5 U BioTherm Star Polymerase (Genecraft, Lüdingshausen, Deutschland)] eingesetzt. Amplifiziert wurde mit folgenden Zyklen:

| | |
|---|---|
| Zyklus 1 | 95 ˚ C, 10 min |
| Zyklus 2 x 30 | 95 ˚ C, 1 min |
| | 56 ˚ C, 1 min |
| | 72 ˚ C, 2 min |

Das Amplifizierunssignal wurde in einer Nest-PCR mit den Oligonukleotiden SEQIDNo 109 und SEQIDNo 111 verstärkt.

Anschließend wurde die Amplifizierungsreaktion auf 4 ˚ C abgekühlt und gesamt auf ein 1% Agarose Gel aufgetragen. Es resultierte ein spezifische Fragment einer Größe von ungefähr 1000 bp. Nach der Elution aus dem Gel wurde das Fragment in den pCR2.1 Vektor (Invitrogen, Karlsruhe, Deutschland) ligiert.

Die Sequenzanalyse des das Fragment enthaltenden Plasmids lieferte Informationen über die 5' und 3' flankierende Sequenzen. Somit besitzt das DNA Segment, das für das Protein kodiert, eine gesamt Länge von 1044 bp, das mit einem StopKodon endet (SEQIDNo 13), und weist eine offene Leserrahmen von 347 Aminosäuren (SEQIDNo 5) auf.

Genomische DNA der Microbacterium sp. Zellen wurde als Matrize für die Generierung der das Protein kodierenden Volllängen-DNA in einer Polymerase Kettenreaktion mit Hilfe von GC-Rich PCR System (Roche, Mannheim, Deutschland) und 30 pMol Oligonikleotiden SEQIDNo 123, bzw. SEQIDNo 124 mit 30 pMol Olidonukleotid SEQIDNo 125 und Temperatur-Zyklen:

| | |
|---|---|
| Zyklus 1 | 95 ° C, 3 min |
| Zyklus 2 x 30 | 95 ° C, 30 sec |
| | 59 ° C, 30 sec |
| | 72 ° C, 45 sec |

verwendet.

Das resultierende PCR-Produkt wurde nach der Reinigung über 1% Agarose Gel mit Nde I und Hind III, bzw. mit Sph I und Hind III behandelt und in das mit den gleichen Endonukleasen behandelten Rückgrad des Vektors pET21a (Novagen, Madison, USA), bzw. pQE32 (Qiagen, Hilden, Deutschland) ligiert. Nach der Transformation von 2 μl des Ligationsansatzes in E.coli Top10F' Zellen wurden Plasmid-DNA's Ampicillin-resistenter Kolonien mittels einer Restriktionsanalyse mit Endonukleasen Nde I, bzw. Sph I und Hind III auf die Richtigkeit der erfolgten Ligation überprüft. Die DNA der für das Insert positiven Vektoren wurde in den Expressionsstamm BL21 Star (Invitrogen), bzw. RB791 (E.coli genetic Stock, Yale, USA) transformiert.

*Beispiel 6:*

*Expression von rekombinanten Alkoholdehydrogenasen / Reduktasen in E.coli*

Die mit dem Expressionskonstrukt transformierte Escherichia coli Stämme BL21 Star (Invitrogen, Karlsruhe, Deutschland), bzw. RB791 (E.coli genteic stock, Yale, USA) wurden in 200 ml LB-Medium (1% Tryptone, 0.5% Hefeextrakt, 1% NaCl) mit Ampicillin (50 μg / ml), bzw. Carbenicillin (50 μg / ml) kultiviert, bis eine Optische Dichte gemessen bei 550 nm von 0.5 erreicht wurde. Die Expression von rekombinantem Protein wurde durch Zugabe von Isopropylthiogalaktosid (IPTG) in einer Konzentration von 0.1 mM induziert. Nach 8 Stunden, bzw. nach 16 Stunden Induktion bei 25 ° C und 220 rpm wurden die Zellen geerntet und bei -20 ° C eingefroren. Für den Aktivitätstest wurden 10 mg Zellen mit 500 μl 100 mM TEA Puffer pH 7.0 und 500 μl Glasperlen versetzt und 10 min mittels einer Kugelmühle aufgeschlossen. Das erhaltene Lysat wurde dann verdünnt für die entsprechenden Messungen eingesetzt. Aktivitätstest setzte sich wie folgt zusammen: 870 μl 100 mM TEA Puffer pH 7.0, 160 μg NAD(P)H, 10 μl verdünntes Zelllysat. Reaktion wurde gestartet bei der Zugabe von 100 μl einer 100 mM Substratlösung zum dem Reaktionsgemisch.

| | Expressionsvektor | Expressionsstamm | Substrat | Aktivität U / g |
|---|---|---|---|---|
| SEQ ID No 1 | pET21a | BL21 Star | Acetophenon | 4700 U/g |
| SEQ ID No 2 | pET21a | BL21 Star | 2-Butanon | 1900 U/g |
| SEQ ID No 3 | pQE70 | RB791 | CLAEE | 5220 U/g |
| SEQ ID No 4 | pET21a | BL21 Star | CLAEE | 8300 U/g |
| SEQ ID No 5 | pET21a | BL21 Star | 2-Octanon | 8000 U/g |
| SEQ ID No 6 | pQE70 | RB791 | 2-Octanon | 1600 U/g |
| SEQ ID No 7 | pET21a | BL21 Star | | |
| SEQ ID No 8 | pET21a | BL21 Star | CLAEE | 7000 U/g |

*Beispiel 7:*

*Charakterisierung der rekombinanten Oxidoreduktasen*

*7a: pH-Optimum*

Es wurden die in Tabelle 4 aufgeführten Puffer hergestellt. Die Konzentration der jeweiligen Pufferkomponenten betrug jeweils 50 mM.

Tabelle 4

| PH-Wert | Puffersystem | pH-Wert | Puffersystem |
|---|---|---|---|
| 4 | Na-acetat/Essigsäure | 7,5 | $KH_2PO_4/K_2PO_4$ |
| 4,5 | Na-acetat/Essigsäure | 8 | $KH_2PO_4/K_2PO_4$ |
| 5 | Na-acetat/Essigsäure | 8,5 | $KH_2PO_4/K_2PO_4$ |
| 5,5 | $KH_2PO_4/K_2PO_4$ | 9 | Glycin/NaOH |
| 6 | $KH_2PO_4/K_2PO_4$ | 9,5 | Glycin/NaOH |
| 6,5 | $KH_2PO_4/K_2PO_4$ | 10 | Glycin/NaOH |
| 7 | $KH_2PO_4/K_2PO_4$ | 11 | Glycin/NaOH |

Meßansatz (30°C)-pH Optimum Reduktion:

| | | |
|---|---|---|
| 870 | µl | der jeweils in Tabelle 3 genannten Puffersysteme |
| 20 | µl | NAD(P)H 10 mM |
| 10 | µl | Enzym verdünnt |

Es wurde etwa 2 bis 3 min inkubiert, danach erfolgte die Zugabe von

100 µl Substratlösung (100mM)

Als Substrat wurden je nach Oxidoreduktase 2-Butanon oder 2-Oktanon eingesetzt.

Die Reaktion wurde 1 min bei 340 nm verfolgt. Zur Bestimmung des pH-Optimums wurde die enzymatische Reaktion in dem jeweiligen in Tabelle 4 aufgeführten Puffer bestimmt. Zur Bestimmung des pH-Optimums für die Oxidationsreaktion wurde als Cofaktor NAD(P) und als Substrat 2-Propanol oder 2-Oktanol eingesetzt.

In Tabelle 5 sind die Ergebnisse für die erfindungsgemäßen Oxidoreduktasen zusammengestellt.

Tabelle 5:

| DSMZ Nr. | Mikrorganismus | pH-opt red | pH-opt ox |
|---|---|---|---|
| 70825 | Rhodotorula mucilaginosa | 7-8 | 8,0-9,5 |
| 3316 | Pichia farinosa | 5-6 | 7-11 |
| 70647 | Candida nemodendra | 6 | 10-11 |
| 3651 | Pichia stipidis | 5,5-6,5 | 6,5-7,5 |
| 70391 | Pichia trehalophila | 7-7,5 | 7-8 |
| 5576 | Leuconostoc carnosum | 5,0-6 | 6,5-9,5 |
| 20028 | Microbacterium spec. | 6,5-7,5 | 7,5-8,5 |
| 43570 | Gordonia rubripertincta | 5 | 7,5-9,5 |

*7b: pH-Stabilität*

Die Bestimmung der Aktivität der rekombinanten Oxidoreduktasen wurde durch Lagerung in den in Tabelle 4 genannten Puffersystemen untersucht. Dazu wurden die verschiedene Puffer (50mM) im Bereich von pH 4 bis 11 angesetzt und die gemäß Beispiel 4 hergestellte Oxidoreduktase damit verdünnt. Nach 30, 60 und 120 min Inkubation wurden aus dem Ansatz 10 µl entnommen und im Aktivitätstest gemäß Beispiel 1 eingesetzt.

Ausgangswert ist dabei der Messwert, den man unmittelbar nach Verdünnung (1:20) des Enzyms in Kaliumphosphatpuffer 50 mM pH = 7.0 erhielt. Dieser Wert entsprach unter den vorgegeben Bedingungen einer Extinktionsänderung von etwa 0,70 /min und wurde als 100%-Wert gesetzt und alle folgenden Messwerte wurden zu diesem Wert ins Verhältnis gesetzt.

In Tabelle 6 sind für die erfindungsgemäßen Oxidoreduktasen die pH-Bereiche zusammengestellt indem die Enzyme bei 120 min Inkubation nicht weniger als 50% der Ausgangsaktivität aufwiesen.

Tabelle 6:

| DSMZ Nr. | Mikrorganismus | pH-Bereich Stabilität |
|---|---|---|
| 70825 | Rhodotorula mucilaginosa | 5,5-9,5 |
| 3316 | Pichia farinosa | 5,5-10,0 |
| 70647 | Candida nemodendra | 6,5-9,5 |
| 3651 | Pichia stipidis | 6,0-7,0 |
| 70391 | Pichia trehalophila | 6,0-8,0 |
| 5576 | Leuconostoc carnosum | 4,5-9,5 |
| 20028 | Microbacterium spec. | 5,0-9,5 |
| 43570 | Gordonia rubripertincta | 4,5-10 |

*7c: Temperaturoptimum*

Zur Bestimmung der optimalen Testtemperatur wurde die Enzymaktivität für die erfindungsgemäßen Oxidoreduktasen im Temperaturbereich von 15°C bis 70°C im Standardmeßansatz gemessen.

Die ermittelten Temperaturoptima sind in Tabelle 7 zusammengefasst:

Tabelle 7

| DSMZ Nr. | Mikrorganismus | Topt |
|---|---|---|
| 70825 | Rhodotorula mucilaginosa | 50°C |
| 3316 | Pichia farinosa | 40°C |
| 70647 | Candida nemodendra | 65°C |
| 3651 | Pichia stipidis | 40°C |
| 70391 | Pichia trehalophila | n.b. |
| 5576 | Leuconostoc carnosum | 60°C |
| 20028 | Microbacterium spec. | 60°C |
| 43570 | Gordonia rubripertincta | 45-55 °C |

*7d: Temperaturstabilität*

In analoger Weise wie unter Beispiel 5c beschrieben wurde die Temperaturstabilität für den Bereich von 15°C bis 70°C bestimmt. Dazu wurde jeweils eine Verdünnung der erfindungsgemäßen Oxidoreduktasen für 60 min und 180 min bei der jeweiligen Temperatur inkubiert und anschließend bei 30°C mit dem obigen Testansatz gemessen. In Tabelle 8 sind für die erfindungsgemäßen Oxidoreduktasen die Temperaturbereiche zusammengestellt indem die Enzyme bei 120 min Inkubation nicht weniger als 50% der Ausgangsaktivität aufwiesen.

Tabelle 8

| DSMZ Nr. | Mikrorganismus | Temperaturstabilität |
|---|---|---|
| 70825 | Rhodotorula mucilaginosa | 15-35°C |
| 3316 | Pichia farinosa | 15-25°C |
| 70647 | Candida nemodendra | 15- 35°C |

(fortgesetzt)

| DSMZ Nr. | Mikrorganismus | Temperaturstabilität |
|---|---|---|
| 3651 | Pichia stipidis | 15-35˚C |
| 70391 | Pichia trehalophila | 15- 35˚C |
| 5576 | Leuconostoc carnosum | 15-35˚C |
| 20028 | Microbacterium spec. | 15-60˚C |
| 43570 | Gordonia rubripertincta | 15- 55˚C |

*7e: Substratspektrum*

Das Substratspektrum der erfindungsgemäßen Oxidoreduktasen wurde durch Messung der Enzymaktivität für Reduktion und Oxidation mit einer Reihe von Ketonen und Alkoholen bestimmt. Dazu wurde der Standard Messansatz gemäß Beispiel 1 mit unterschiedlichen Substraten verwendet.

Die Aktivität mit Methylacetoacetat wurde für alle Enzyme gleich 100% gesetzt und alle anderen Substrate wurden dazu ins Verhältnis gesetzt.

Tabelle 9: Substratspektren Reduktion

| Substrat | Rhodotorula mucilaginosa SEQ ID NO1 | Pichia farinosa SEQ ID NO2 | Pichia stipidis SEQID NO3 | Leuconostoc carnosum SEQID NO4 | Micro-bacterium spec. SEQID NO5 | Gordonia rubri-pertincta SEQID NO6 |
|---|---|---|---|---|---|---|
| 1-Phenyl-2-propanon | 66% | 10% | 30% | 13% | 80% | 82 % |
| Phenacyl-Chlorid | 36% | 130% | 9% | 37% | < 2% | 7% |
| Acetophenon | 12% | 195% | 32% | 28% | 52% | 23 % |
| Acetonaphton | n.b. | 25% | 84% | n.b. | 125% | 68 % |
| Butyrophenon | 0% | 0% | 0% | 0% | 0% | 0% |
| 2-Octanon | 71% | 20% | 27% | 28% | 227% | 75 % |
| 3-Octanon | 29% | 10% | 40% | 18% | 47% | 52 % |
| 2-Butanon | 190% | 65% | 49% | 36% | 4% | 14% |
| Ethyl-2-oxovaleriat | 4% | 85% | 60% | 25% | < 2% | 23 % |
| Ethyl-2-oxo-4-phenyl buttersäure | 4% | 35% | 16% | 10% | < 2% | 18 % |
| Ethylpyruvat | 60% | 560% | 148% | 122% | 480% | 160 % |
| Ethylphenyl-glyoxylat | 8% | 35% | 3% | 4% | < 2% | 11% |
| Ethyl-4-chloro acotoacetat | 79% | 70% | 100% | 80% | 110% | 110% |
| Methyl acetoacetat | 100% | 100% | 100% | 100% | 100% | 100% |
| Ethyl-3-oxovaleriat | 60% | 45% | 73% | 30% | < 2% | 56 % |
| Aceton | 82% | 55% | 100% | 28% | < 2% | 7% |

*7f: Stabilität im wässrig/organischen Zwei -Phasensystem*

Die Stabilität der neuartigen Oxidoreduktasen in wässrig/ organischen Zweiphasensystemen wurde untersucht, indem die in Beispiel 6 gewonnenen Lysate (aus rekombinanter Expression) in einem für die jeweilige Oxidoreduktase geeigneten, wässrigem Puffer verdünnt wurden (ca. 10 Units/ml Puffer). Zu der im Puffer verdünnten Oxidoreduktase wurde dann das selbe Volumen eines organischen, nicht wassermischbaren Lösungsmittels gegeben und der Ansatz bei Raumtemperatur unter ständiger Durchmischung (Thermomixer mit 170 rpm) inkubiert. Nach 24 h Inkubation wurden jeweils aus der wässrigen Phase 10 µl entnommen und für die Bestimmung der Enzymaktivität im Standardtestansatz (Kaliumphosphatpuffer (KPP) 100 mM, pH = 7.0, 0,2 mM NAD(P)H, 10 mM Substrat) eingesetzt. Auch hier wurde der Ausgangswert direkt nach Verdünnung im Puffer gleich 100% gesetzt und alle weiteren Werte zu diesem ins Verhältnis gesetzt.

Tabelle 9: Enzymaktivität nach 24 h Inkubation im wässrig organischen Zwei-Phasensystem

| System | Puffer | Butyl-acetat | Diethyl-ether | MTBE | Diisopro pyl-ether | Heptan | Cyclo-hexan |
|---|---|---|---|---|---|---|---|
| Rhodotorula mucilaginosa SEQ ID No 1 | 100% | 80-100% | 80-100% | 80-100% | 80-100% | 80-100% | 80-100% |
| Pichia farinosa SEQ ID No 2 | 100% | 40-60 | 60-80% | 80-100% | 40-60% | 40-60 | 40-60% |
| Candida nemodendra SEQ ID No 8 | 100% | 80-100% | 80-100% | 80-100% | 80-100% | 80-100% | 80-100% |
| Pichia stipidis SEQ ID No 3 | 100% | 40-60% | n.b. | 20-50% | 60-80% | 80-100% | 40-60% |
| Leuconostoc carnosum SEQ ID No 4 | 100% | 80-100% | 80-100% | 80-100% | 80-100% | 80-100% | 80-100% |
| Microbacterium spec. SEQ ID No 5 | 100% | 80-100% | 80-100% | 80-100% | 80-100% | 80-100% | 80-100% |
| Gordonia rubripertincta SEQ ID No 6 | 100% | 80-100% | 80-100% | 80-100% | 80-100% | 80-100% | 80-100% |
| MTBE = Methyl-tert-butylether | | | | | | | |

Tabelle 10: Substratspektren Oxidation

| Substrat | Rhodotorula mucilaginosa SEQ ID NO1 | Pichia farinosa SEQ ID NO2 | Pichia stipidis SEQID NO3 | Leuconostoc carnosum SEQID NO4 | Micro-bacterium spec. SEQID NO5 | Gordonia rubri-pertincta SEQID NO6 |
|---|---|---|---|---|---|---|
| S-2-Oktanol | 100% | 0% | 100% | 0% | 100% | 100% |
| R-2-Oktanol | 0% | 100% | 0% | 100% | 0% | 0% |
| S-2-Butanol | 266% | 100% | 237% | 56% | 5% | 45 % |
| R-2-Butanol | 60% | 340% | 74% | 178% | 0% | 17% |
| S-Phenyl-2-propanol | 200% | 0% | 26% | 0% | 10% | 0% |
| R-Phenyl-2-propanol | 0% | 0% | 0% | 6% | 0% | 0% |

(fortgesetzt)

| Substrat | Rhodotorula mucilaginosa SEQ ID NO1 | Pichia farinosa SEQ ID NO2 | Pichia stipidis SEQID NO3 | Leuconostoc carnosum SEQID NO4 | Micro-bacterium spec. SEQID NO5 | Gordonia rubri-pertincta SEQID NO6 |
|---|---|---|---|---|---|---|
| Ethyl-(S)-4-chloro-3-hydroxy-butyrate | 0% | 0% | 0% | 0% | 0% | 0% |
| Ethyl-(R)-4-chloro-3-hydroxy-butyrate | 0% | 0% | 0% | 0% | 10% | 0% |
| 2-Propanol | 180% | 180% | 218% | 67% | < 1% | 27% |
| Cyclohexanol | 26% | 120% | 0% | n.b. | n.b. | 7% |

*Beispiel 8: Präparative Umsetzungen*

*8a: Synthese von Methyl-(3S)-3-hydroxypentanoate mit Oxidoreduktase aus Rhodotorula mucilaginosa*

Für den präperativen Ansatz wurde ein Gemisch aus 25 ml Puffer (100mM TEA, pH = 7, 1 mM $ZnCl_2$, 10% Glycerin), 375 ml 4-Methyl-2-pentanol, 100 ml Methyl 3-oxopentanoate, 100 mg NAD und 37 kU rekombinante Oxidoreduktase aus Rhodotorula mucillaginosa DSMZ 70825 für 24 h bei Raumtemperatur unter ständiger Durchmischung inkubiert. Nach 24 h waren 97% des eingesetzten Methyl 3-oxopentanoate zu Methyl-(3S)-3-hydroxypentanoate reduziert. Die das Produkt enthaltende 4-Methyl-2-pentanolphase wurde anschließend von der wässrigen Phase abgetrennt, filtriert und das Produkt Methyl-(3S)-3-hydroxypentanoate durch Destillation gewonnen.

Auf diese Weise wurde das Produkt Methyl-(3S)-3-hydroxypentanoate in einer Reinheit von > 99% und mit einem Enantiomerenüberschuss von >99,5% in hoher Ausbeute gewonnen.

*8b: Synthese von (2R)-1-chloropropan-2-ol mit Oxidoreduktase aus Pichia farinosa*

Für die Umsetzung wurde ein Gemisch aus 80 ml Puffer (100mM TEA, pH = 7, 1 mM $MgCl_2$, 10% Glycerin), 15 ml 2-Propanol, 5 ml Chloraceton, 10 mg NAD und 2 kU rekombinante Oxidoreduktase aus Pichia farinosa DSMZ 3316 für 24 h bei Raumtemperatur unter ständiger Durchmischung inkubiert. Nach 24 h waren das eingesetzte Chloraceton vollständig zu (2R)-1-chloropropan-2-ol reduziert. Das Reaktionsgemisch wurde anschließend mit Ethylacetat extrahiert, das Lösungsmittel mittels Rotationsverdampfer entfernt und das Rohprodukt wurde gewonnen. Das auf diese Weise hergestellte (2R)-1-chloropropan-2-ol weist einen mit einem Enantiomerenüberschuss von >99% auf.

*8c: Synthese von (R)-2-Chlor-1-(3-chlorphenyl)ethan-1-ol mit Oxidoreduktase aus Pichia stipidis*

Für die Umsetzung wurde ein Gemisch aus 20 ml Puffer (100 mM Kaliumphosphat , pH = 8,5, 1 mM $MgCl_2$, 10% Glycerin), 20 g 2-Chlor-1-(3-chlorphenyl)ethan-1-on gelöst in 80 ml 4-Methyl-2-pentanol, 10 mg NAD und 20 000 U rekombinante Oxidoreduktase aus Pichia stipidis DSMZ 3651 für 24 h bei Raumtemperatur unter ständiger Durchmischung inkubiert. Nach 24 h waren mehr als 99% des eingesetzten 2-Chlor-1-(3-chlorphenyl)ethan-1-on's reduziert. Die das Produkt enthaltende 4-Methyl-2-pentanolphase wurde anschließend von der wässrigen Phase abgetrennt, filtriert und das Produkt (R)-2-Chlor-1-(3-chlorphenyl)ethan-1-ol durch Destillation gewonnen.

Auf diese Weise wurde das Produkt (R)-2-Chlor-1-(3-chlorphenyl)ethan-1-ol in einer Reinheit von > 98% und mit einem Enantiomerenüberschuss von >99,9% in hoher Ausbeute gewonnen.

*8d: Synthese von Ethyl-(S)-4-chlor-3-hydroxybuttersäure mit Oxidoreduktase aus Leuconostoc carnosum*

Für die Umsetzung wurde ein Gemisch aus 8 mL Puffer (100mM TEA, pH = 7, 1 mM $MgCl_2$), 24 ml Isopropanol, 8 ml Ethyl-4-Chloracetoacetat, 2 mg NADP und 6,7 kU (= 6ml) rekombinante Oxidoreduktase aus Leuconostoc carnosum DSMZ 5576 für 24 h bei Raumtemperatur unter ständiger Durchmischung inkubiert. Nach 24 h waren mehr als 99% des eingesetzten Ethyl-4-chloracetoacetat zu Ethyl-(S)-4-chlor-3-hydroxybuttersäure reduziert. Das Reaktionsgemisch wurde aufgearbeitet indem zuerst das 2-Propanol mittels Rotationsverdampfer entfernt wurde. Das Reaktionsgemisch wurde anschließend mit Ethylacetat extrahiert, das Lösungsmittel mittels Rotationsverdampfer entfernt und das Rohprodukt gewonnen. Das auf diese Weise gewonnene Rohprodukt Ethyl-(S)-4-chlor-3-hydroxybuttersäure_wies einen Enantiomerenüberschuss von > 99,5% auf.

*8e: Synthese von (1S)-1-[3,5-bis(trifluoromethyl)phenyl]ethan-1-ol mit Oxidoreduktase aus Microbacterium spec.*

Für die Umsetzung wurde ein Gemisch aus 1 mL Puffer (100mM TEA, pH = 7, 10%Glycerin, 1 mM $ZnCl_2$), 3 ml 4-Methyl-2-pentanol, 1 ml 1-[3,5 bis-(trifluoro-methyl)phenyl]ethan-1-on, 2 mg NAD und 0,7 kU rekombinante Oxidoreduktase aus Microbacterium spec. DSMZ 20028 für 24 h bei Raumtemperatur unter ständiger Durchmischung inkubiert. Nach 24 h waren mehr als 90% des eingesetzten 1-[3,5 bis-(trifluoro-methyl)phenyl]ethan-1-on zu (1S)-1-[3,5-bis(trifluoromethyl)phenyl]ethan-1-ol_reduziert. Die das Produkt enthaltende 4-Methyl-2-pentanolphase wurde anschließend von der wässrigen Phase abgetrennt, filtriert und das Produkt (1S)-1-[3,5-bis(trifluoromethyl)phenyl]ethan-1-ol durch Destillation gewonnen.

Das auf diese Weise gewonnene Rohprodukt (1S)-1-[3,5-bis(trifluoromethyl)phenyl]ethan-1-on wies einen Enantiomerenüberschuss von > 99,5% auf.

**Patentansprüche**

1. Verfahren zur enantioselektiven enzymatischen Reduktion einer Ketoverbindung zur entsprechenden chiralen Hydroxyverbindung, wobei die Ketoverbindung in Gegenwart eines Co-Faktors mit einer Oxidoreduktase reduziert wird, **dadurch gekennzeichnet, dass** eine Oxidoreduktase eingesetzt wird, welche ausgewählt ist aus der Gruppe, bestehend aus den Oxidoreduktasen, für welche

(a) die Nukleinsäuresequenz SEQ ID NO:11, die Nukleinsäuresequenz SEQ ID NO:15 oder die Nukleinsäuresequenz SEQ ID NO:130 codiert, oder
(b) eine Nukleinsäuresequenz codiert, deren komplementärer Strang mit der in (a) genannten Nukleinsäuresequenzen unter hochstringenten Bedingungen hybridisiert.

**2.** Verfahren nach Anspruch 1, **dadurch gekennzeichnet, dass** die Ketoverbindung die allgemeine Formel I

$$R_1\text{-}C(O)\text{-}R_2 \qquad (I)$$

aufweist, in der R1 für einen der Reste

1) $-(C_1-C_{20})$-Alkyl, worin Alkyl geradkettig oder verzweigtkettig ist,
2) $-(C_2-C_{20})$-Alkenyl, worin Alkenyl geradkettig oder verzweigtkettig ist und gegebenenfalls bis zu vier Doppelbindungen enthält,
3) $-(C_2-C_{20})$-Alkinyl, worin Alkinyl geradkettig oder verzweigtkettig ist und gegebenenfalls bis zu vier Dreifachbindungen enthält,
4) $-(C_6-C_{14})$-Aryl,
5) $-(C_1-C_8)$-Alkyl-$(C_6-C_{14})$-Aryl,
6) $-(C_5-C_{14})$-Heterocyclus, der unsubstituiert oder ein-, zwei- oder dreifach durch -OH, Halogen, $-NO_2$ und/oder $-NH_2$ substituiert ist, oder
7) $-(C_3-C_7)$-Cycloalkyl,
steht, wobei die oben unter 1) bis 7) genannten Reste unsubstituiert sind oder unabhängig voneinander ein-, zwei- oder dreifach durch -OH, Halogen, $-NO_2$ und/oder $-NH_2$ substituiert sind,
und $R_2$ für einen der Reste
8) $-(C_1-C_6)$-Alkyl, worin Alkyl geradkettig oder verzweigtkettig ist,
9) $-(C_2-C_6)$-Alkenyl, worin Alkenyl geradkettig oder verzweigtkettig ist und gegebenenfalls bis zu drei Doppelbindungen enthält,
10) $-(C_2-C_6)$-Alkinyl, worin Alkinyl geradkettig oder verzweigtkettig ist und gegebenenfalls zwei Dreifachbindungen enthält, oder
11) $-(C_1-C_{10})$-Alkyl-C(O)-O-$(C_1-C_6)$-Alkyl, worin Alkyl gerade oder verzweigtkettig ist und unsubstituiert ist oder ein-, zwei- oder dreifach durch -OH, Halogen, $-NO_2$ und/oder - $NH_2$ substituiert ist,
steht, wobei die oben unter 8) bis 11) genannten Reste unsubstituiert sind oder unabhängig voneinander ein-, zwei- oder dreifach durch -OH, Halogen, $-NO_2$ und/oder

- $NH_2$ substituiert sind,

stehen.

**3.** Klonierungsvektor, enthaltend eine oder mehrere Nukleinsäuresequenzen, welche für die Oxidoreduktase gemäß SEQ ID NO:3, SEQ ID NO:7 oder SEQ ID NO:129 codieren.

**4.** Expressionsvektor, der sich in einer Bakterien-, Insekten-, Pflanzen- oder Säugetierzelle in vitro befindet und eine Nukleinsäuresequenz enthält, welche für die Oxidoreduktase gemäß SEQ ID NO:3, SEQ ID NO:7 oder SEQ ID NO:129 codiert und mit einer Expressionskontrollsequenz verbunden ist.

**5.** Rekombinante Wirtszelle, die eine Bakterien-, Insekten-, Pflanzen- oder Säugetierzelle ist und mit einem Expressionsvektor gemäß Anspruch 4 transformiert oder transfektiert wurde.

**6.** Oxidoreduktase zur Verwendung in einem Verfahren gemäß Anspruch 1 oder 2, **dadurch gekennzeichnet, dass** sie von der Nukleinsäuresequenz codiert wird, die ausgewählt ist aus der Gruppe bestehend aus SEQ ID NO:11, SEQ ID NO:15, SEQ ID NO:130 und einer Nukleinsäuresequenz, deren komplementärer Strang mit der Sequenz SEQ ID NO:11, SEQ ID NO:15 oder SEQ ID NO:130 unter hochstringenten Bedingungen hybridisiert.

7. Verfahren zur enantioselektiven enzymatischen Reduktion einer Ketoverbindung zur entsprechenden chiralen Hydroxyverbindung, wobei die Ketoverbindung mit einer Oxidoreduktase in Gegenwart eines Co-Faktors reduziert wird, **dadurch gekennzeichnet, dass**
eine Oxidoreduktase eingesetzt wird, die die Aminosäuresequenz SEQ ID NO:3 oder eine Aminosäuresequenz aufweist, bei welcher mindestens 50% der Aminosäuren identisch sind mit den Aminosäuren der Aminosäuresequenz SEQ ID NO:7 oder mindestens 72% der Aminosäuren identisch sind mit den Aminosäuren der Aminosäuresequenz SEQ ID NO:129.

Europäisches Patentamt
European Patent Office
Office européen des brevets

# EUROPÄISCHER RECHERCHENBERICHT

Nummer der Anmeldung
EP 11 18 9707

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | WO 2004/111083 A2 (JUELICH ENZYME PRODUCTS GMBH [DE]; GUPTA ANTJE [DE]; ZIMMER ANKE [DE];) 23. Dezember 2004 (2004-12-23)<br>* Seite 9, Zeile 13 - Seite 11, Zeile 3; Ansprüche; Sequenzen 8, 9 *<br>----- | 1-7 | INV.<br>C12P7/02<br>C12N9/02<br>C12P41/00 |
| A | EP 0 645 453 A2 (DAICEL CHEM [JP])<br>29. März 1995 (1995-03-29)<br>* Ansprüche; Beispiele 2-6 *<br>----- | 1-7 | |
| A | DATABASE UniProt [Online]<br><br>1. Juni 1998 (1998-06-01),<br>"SubName: Full=SADH;",<br>XP002668450,<br>gefunden im EBI accession no. UNIPROT:O42703<br>Database accession no. O42703<br>* Sequenz *<br>----- | 1-7 | |
| A | DATABASE EMBL [Online]<br><br>28. Januar 1998 (1998-01-28),<br>"Candida parapsilosis gene for SADH, complete cds.",<br>XP002668451,<br>gefunden im EBI accession no. EM_FUN:AB010636<br>Database accession no. AB010636<br>* Sequenz *<br>----- | 1-7 | RECHERCHIERTE SACHGEBIETE (IPC)<br>C12P<br>C12N |

1 Der vorliegende Recherchenbericht wurde für alle Patentansprüche erstellt

| Recherchenort | Abschlußdatum der Recherche | Prüfer |
|---|---|---|
| München | 31. Januar 2012 | Huber, Angelika |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.....................................................
& : Mitglied der gleichen Patentfamilie, übereinstimmendes Dokument

EPO FORM 1503 03.82 (P04C03)

Europäisches Patentamt
European Patent Office
Office européen des brevets

Nummer der Anmeldung

EP 11 18 9707

## GEBÜHRENPFLICHTIGE PATENTANSPRÜCHE

Die vorliegende europäische Patentanmeldung enthielt bei ihrer Einreichung Patentansprüche, für die eine Zahlung fällig war.

☐ Nur ein Teil der Anspruchsgebühren wurde innerhalb der vorgeschriebenen Frist entrichtet. Der vorliegende europäische Recherchenbericht wurde für jene Patentansprüche erstellt, für die keine Zahlung fällig war, sowie für die Patentansprüche, für die Anspruchsgebühren entrichtet wurden, nämlich Patentansprüche:

☐ Keine der Anspruchsgebühren wurde innerhalb der vorgeschriebenen Frist entrichtet. Der vorliegende europäische Recherchenbericht wurde für die Patentansprüche erstellt, für die keine Zahlung fällig war.

## MANGELNDE EINHEITLICHKEIT DER ERFINDUNG

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

Siehe Ergänzungsblatt B

☐ Alle weiteren Recherchengebühren wurden innerhalb der gesetzten Frist entrichtet. Der vorliegende europäische Recherchenbericht wurde für alle Patentansprüche erstellt.

☒ Da für alle recherchierbaren Ansprüche die Recherche ohne einen Arbeitsaufwand durchgeführt werden konnte, der eine zusätzliche Recherchengebühr gerechtfertigt hätte, hat die Recherchenabteilung nicht zur Zahlung einer solchen Gebühr aufgefordert.

☐ Nur ein Teil der weiteren Recherchengebühren wurde innerhalb der gesetzten Frist entrichtet. Der vorliegende europäische Recherchenbericht wurde für die Teile der Anmeldung erstellt, die sich auf Erfindungen beziehen, für die Recherchengebühren entrichtet worden sind, nämlich Patentansprüche:

☐ Keine der weiteren Recherchengebühren wurde innerhalb der gesetzten Frist entrichtet. Der vorliegende europäische Recherchenbericht wurde für die Teile der Anmeldung erstellt, die sich auf die zuerst in den Patentansprüchen erwähnte Erfindung beziehen, nämlich Patentansprüche:

☐ Der vorliegende ergänzende europäische Recherchenbericht wurde für die Teile der Anmeldung erstellt, die sich auf die zuerst in den Patentansprüchen erwähnte Erfindung beziehen (Regel 164 (1) EPÜ).

Europäisches Patentamt
European Patent Office
Office européen des brevets

# MANGELNDE EINHEITLICHKEIT DER ERFINDUNG ERGÄNZUNGSBLATT B

Nummer der Anmeldung
EP 11 18 9707

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

1. Ansprüche: 1-7(teilweise)

Verfahren zur enantioselektiven Reduktion einer Ketoverbindung zur entsprechenden chiralen Hydroxyverbindung, wobei eine Oxidoreduktase eingesetzt wird, für welche die Nukleinsäuresequenz SEQ ID NO: 11 kodiert oder eine Nukleinsäuresequenz deren komplementärer Strang mit der genannten Nukleinsäuresequenz unter hochstringenten Bedingungen hybrisisiert; Vektor und Wirtszelle enthaltend eine Nukleinsäuresequenz die für eine Oxidoreduktase gemäss SEQ ID NO:3 kodiert; von dieser Nukleinsäure kodierte Oxidoreduktase zur Verwendung in dem genannten Verfahren; Verfahren zur enantioselektiven Reduktion einer Ketoverbindung zur entsprechenden chiralen Hydroxyverbindung, wobei eine Oxidoreduktase eingesetzt wird, die die der Aminosäuresequenz SEQ ID NO: 3 aufweist.

---

2. Ansprüche: 1-7(teilweise)

Verfahren zur enantioselektiven Reduktion einer Ketoverbindung zur entsprechenden chiralen Hydroxyverbindung, wobei eine Oxidoreduktase eingesetzt wird, für welche die Nukleinsäuresequenz SEQ ID NO: 15 kodiert oder eine Nukleinsäuresequenz dren komplementärer Strang mit der genannten Nukleinsäuresequenz unter hochstringenten Bedingungen hybrisisiert; Vektor und Wirtszelle enthaltend eine Nukleinsäuresequenz die für eine Oxidoreduktase gemäss SEQ ID NO: 7 kodiert; von dieser Nukleinsäure kodierte Oxidoreduktase zur Verwendung in dem genannten Verfahren; Verfahren zur enantioselektiven Reduktion einer Ketoverbindung zur entsprechenden chiralen Hydroxyverbindung, wobei eine Oxidoreduktase eingesetzt wird, bei welcher mindestens 50% der Aminosäuren identisch sind mit der Aminosäuresequenz SEQ ID NO: 7.

---

3. Ansprüche: 1-7(teilweise)

Verfahren zur enantioselektiven Reduktion einer Ketoverbindung zur entsprechenden chiralen Hydroxyverbindung, wobei eine Oxidoreduktase eingesetzt wird, für welche die Nukleinsäuresequenz SEQ ID NO: 130 kodiert oder eine Nukleinsäuresequenz dren komplementärer Strang mit der genannten Nukleinsäuresequenz unter hochstringenten Bedingungen hybrisisiert; Vektor und Wirtszelle enthaltend eine Nukleinsäuresequenz die für eine Oxidoreduktase gemäss SEQ ID NO:129 kodiert; von dieser Nukleinsäure kodierte Oxidoreduktase zur Verwendung in dem genannten Verfahren; Verfahren zur enantioselektiven Reduktion einer Ketoverbindung zur entsprechenden chiralen

Europäisches Patentamt
European Patent Office
Office européen des brevets

**MANGELNDE EINHEITLICHKEIT DER ERFINDUNG ERGÄNZUNGSBLATT B**

Nummer der Anmeldung

EP 11 18 9707

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

Hydroxyverbindung, wobei eine Oxidoreduktase eingesetzt wird, die eine Aminosäuresequenz aufweist, bei welcher mindestens 72% der Aminosäuren identisch sind mit der Aminosäuresequenz SEQ ID NO:129.

---

## ANHANG ZUM EUROPÄISCHEN RECHERCHENBERICHT ÜBER DIE EUROPÄISCHE PATENTANMELDUNG NR.

EP 11 18 9707

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

31-01-2012

| Im Recherchenbericht angeführtes Patentdokument | Datum der Veröffentlichung | Mitglied(er) der Patentfamilie | Datum der Veröffentlichung |
|---|---|---|---|
| WO 2004111083 A2 | 23-12-2004 | AT 484518 T | 15-10-2010 |
| | | CA 2529583 A1 | 23-12-2004 |
| | | CN 1839153 A | 27-09-2006 |
| | | DE 10327454 A1 | 20-01-2005 |
| | | DK 1633779 T3 | 31-01-2011 |
| | | EP 1633779 A2 | 15-03-2006 |
| | | ES 2357113 T3 | 18-04-2011 |
| | | HK 1095336 A1 | 27-08-2010 |
| | | JP 4845729 B2 | 28-12-2011 |
| | | JP 2007523617 A | 23-08-2007 |
| | | JP 2011244829 A | 08-12-2011 |
| | | KR 20060030478 A | 10-04-2006 |
| | | PT 1633779 E | 18-01-2011 |
| | | SI 1633779 T1 | 31-03-2011 |
| | | US 2007243594 A1 | 18-10-2007 |
| | | WO 2004111083 A2 | 23-12-2004 |
| EP 0645453 A2 | 29-03-1995 | DE 69434148 D1 | 30-12-2004 |
| | | DE 69434148 T2 | 16-02-2006 |
| | | EP 0645453 A2 | 29-03-1995 |
| | | JP 3574682 B2 | 06-10-2004 |
| | | JP 7231785 A | 05-09-1995 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 10119274
- DE 10327454
- DE 10337401
- DE 10300335
- US 5523223 A
- US 5763236 A
- US 5200335 A
- DE 19610984 A1
- US 05385833 A
- US 5385833 A


### In der Beschreibung aufgeführte Nicht-Patentliteratur


- *Enzyme Microb Technol.,* November 1993, vol. 15 (11), 950-8
- *Biosci. Biotechnol. Biochem.,* 1999, vol. 63 (10), 1721-1729
- *Appl Microbiol Biotechnol.,* 26. April 2003, vol. 62 (4), 380-6
- *J Org Chem.,* 24. Januar 2003, vol. 68 (2), 402-6
- *Acta Crystallogr D Biol Crystallogr.,* Dezember 2000, vol. 56, 1696-8
- *Appl Microbiol Biotechnol.,* 26. Juni 2002, vol. 59 (4-5), 483-7
- *J. Org. Chem.,* 1992, vol. 57, 1532
- **ALTSCHUL et al.** *Proc. Natl. Acd. Sci. USA.,* 1990, vol. 87, 2264-2268
- A model of evolutionary change in Proteins. **DAYHOFF; M.O. ; SCHWARZ, R.M. ; ORCUTT, B.C.** Atlas of Protein Sequence and structure. National Biomedical Research foundation, 1978, vol. 5, 345-352
- **TISHKOV et al.** *J. Biotechnol. Bioeng.,* 1999, vol. 64, 187-193
- **LOWRY et al.** *Journal of Biological Chemistry,* 1951, vol. 193, 265-275
- **PETERSON et al.** *Analytical Biochemistry,* 1979, vol. 100, 201-220